# EUROPEAN PATENT APPLICATION

(11) **EP 4 321 239 A1**
(43) Date of publication of application: **14.02.2024**
(21) Application number: 23165107.6
(22) Date of filing: 29.03.2023
(51) Int. Cl.: B01D 53/88

(54) **AIR PURIFICATION MODULE, AIR PURIFICATION SYSTEM INCLUDING THE SAME, AND METHOD OF PURIFYING AIR**

(30) Priority: 08.08.2022 KR 20220098785
(71) Applicant: Samsung Electronics Co., Ltd., Suwon-si, Gyeonggi-do 16677 (KR)
(72) Inventor: HAM, Dongjin, 16678, Suwon-si, (KR); KIM, Mijong, 16678, Suwon-si, (KR); KOO, Minseok, 16678, Suwon-si, (KR); KWON, Hyukjae, 16678, Suwon-si, (KR); OH, Sehyeong, 16678, Suwon-si, (KR); LEE, Hyun Chul, 16678, Suwon-si, (KR)
(74) Representative: Elkington and Fife LLP

(57) **Abstract**

Provided are an air purification module and an air purification system including the same. The air purification module is configured to purify influent unpurified air and discharge purified air and includes: a catalyst filter including a photocatalyst and an oxidation catalyst; and a light-emitting heat source disposed adjacent to the catalyst filter, wherein the light-emitting heat source irradiates, to the catalyst filter, light for activating the photocatalyst, and provides heat to activate the oxidation catalyst, and a temperature of the light-emitting heat source is higher than a temperature of the unpurified air.

## Description

### FIELD OF THE INVENTION

The disclosure relates to an air purification module, an air purification system including the same, and a method of purifying air.

### BACKGROUND OF THE INVENTION

In order to remove pollutants contained in the air, a method of adsorbing pollutants by using a porous material with a large specific surface area or a method of decomposing pollutants by using a catalyst is generally used.

Here, a catalyst that decomposes pollutants requires extra energy to activate the catalyst.

### SUMMARY OF THE INVENTION

Since unpurified air contains various pollutants, a plurality of catalysts having various operating modes are generally used to remove various pollutants. For example, a photocatalyst activated by light irradiated and an oxidation catalyst activated by heat provided may be used, and a separate light source for activating the photocatalyst and a separate heat source for activating the oxidation catalyst may be additionally disposed. For example, since an air purification apparatus includes a photocatalyst, a light source, an oxidation catalyst, and a heat source separately, the air purification apparatus may have a complex structure, an increased volume, and low energy efficiency.

Accordingly, there is a demand for methods of removing various pollutants by using a plurality of catalysts, simplifying the structure of the air purification apparatus, reducing the volume of the air purification apparatus for miniaturization, and improving the energy efficiency by decreasing power consumption of the air purification apparatus.

Provided is an air purification module having a simplified structure, a compact volume, and improved energy efficiency, by having a new structure.

Provided is an air purification system including the air purification module.

Provided is a new method of purifying air.

Additional aspects will be set forth in part in the description which follows and, in part, will be apparent from the description, or may be learned by practice of the presented embodiments of the disclosure.

According to an aspect of the disclosure, an air purification module is configured to purify influent unpurified air and discharge purified air and includes: a catalyst filter including a photocatalyst and an oxidation catalyst, and a light-emitting heat source disposed adjacent to the catalyst filter. The light-emitting heat source irradiates, to the catalyst filter, light for activating the photocatalyst and provides heat to the catalyst filter to activate the oxidation catalyst therein, and a temperature of the light-emitting heat source is higher than a temperature of the unpurified air.

According to another aspect of the disclosure, an air purification system includes: the air purification module above, an air supply module disposed upstream of air flow, compared to the air purification module, and an air exhaust module disposed downstream of the air flow, compared to the air purification module.

According to another aspect of the disclosure, a method of purifying air includes: providing a light-emitting heat source, and disposing a catalyst filter, which includes a photocatalyst and an oxidation catalyst, adjacent to the light-emitting heat source. The catalyst filter and the light-emitting heat source are configured to purify influent unpurified air and discharge purified air, the light-emitting heat source irradiates, to the catalyst filter, light for activating the photocatalyst and provides heat to the catalyst filter to activate the oxidation catalyst therein, and a temperature of the light-emitting heat source is higher than a temperature of the unpurified air.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a schematic view of an air purification module according to an embodiment;
FIG. 2 is a schematic side view of an air purification module according to another embodiment;
FIG. 3 is a schematic view of an air purification module including other energy sources different from a light-emitting heat source.
FIG. 4 is a schematic view of a catalyst filter according to an embodiment;
FIG. 5 is a schematic view of a catalyst filter according to another embodiment;
FIG. 6 is a cross-sectional view taken along lines A and A' of the catalyst filter of FIG. 4;
FIG. 7 is a schematic view of a catalyst filter according to an embodiment;
FIG. 8 is a front view of an influent surface of the catalyst filter of FIG. 7 where the unpurified air flows in;
FIG. 9 is a front view of an effluent surface of the catalyst filter of FIG. 7 where the purified air flows out;
FIG. 10 is a cross-sectional view taken in the 4-4' direction of the catalyst filter 100 of FIG. 8;
FIG. 11 is an enlarged view of a first area A1 of a horizontal region of FIG. 10;
FIG. 12 is a schematic view of an air purification module including a dust collecting filter according to an embodiment;
FIG. 13 is a schematic side view of an air purification module according to another embodiment;
FIG. 14 is a schematic side view of an air purification module according to another embodiment;
FIG. 15 is a schematic view of an air purification module according to another embodiment;
FIG. 16 is a schematic view of an air purification module according to another embodiment;
FIG. 17 is a schematic view of an air purification module according to another embodiment;
FIG. 18 is a schematic view of an air purification system according to an embodiment;
FIG. 19 is a schematic view of an air purification system according to another embodiment; and
FIG. 20 is a schematic view of an air purification system according to another embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figure, to explain aspects. As used herein, "a", "an," "the," and "at least one" do not denote a limitation of quantity, and are intended to include both the singular and plural, unless the context clearly indicates otherwise. For example, "an element" has the same meaning as "at least one element," unless the context clearly indicates otherwise. As used herein, "/" may be interpreted as "and", or as "or" depending on the context. "Or" means "and/or." As used herein, the term "and/or" includes any and all combinations of one or more of the associated listed items. Expressions such as "at least one of," when preceding a list of elements, modify the entire list of elements and do not modify the individual elements of the list.

The present invention described hereinbelow may have various modifications and various embodiments, example embodiments will be illustrated in the drawings and more fully described. The present invention may, however, should not be construed as limited to the example embodiments set forth herein, and rather, should be understood as covering all modifications, equivalents, or alternatives falling within the scope of the present invention.

The terms used herein is for the purpose of describing particular embodiments only, and is not intended to be limiting the present invention. An expression used in the singular encompasses the expression of the plural, unless it has a clearly different meaning in the context. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

In the drawings, thicknesses may be magnified or exaggerated to clearly illustrate various layers and regions. Like reference numbers may refer to like elements throughout the drawings and the following description. It will be understood that when one element, layer, film, section, sheet, etc. is referred to as being "on" another element, it can be directly on the other element or intervening elements may be present therebetween. Although the terms "first," "second," etc. may be used herein to describe various elements, these elements should not be limited by these terms. These terms are only used to distinguish one element from another element. In the present specification and drawings, components having substantially the same functional features are referred to the same reference numerals, and thus repeated descriptions will be omitted.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10% or 5% of the stated value. Hereinafter, an air purification module, an air purification system including the same, and an air purification method according to embodiments will be described in more detail.

An air purification module according to an embodiment may be configured to purify influent unpurified air and discharge purified air, and include: a catalyst filter including a photocatalyst and an oxidation catalyst; and a light-emitting heat source disposed adjacent to the catalyst filter, wherein the light-emitting heat source may irradiate, to the catalyst filter, light for activating the photocatalyst and provide heat to the catalyst filter to activate the oxidation catalyst therein, and a temperature of the light-emitting heat source may be higher than a temperature of the unpurified air.

The air purification module may include the light-emitting heat source, and since the light-emitting heat source activates the photocatalyst and the oxidation catalyst at the same time, the air purification module may have a simplified structure, a compact volume, and improved energy efficiency, compared to an air purification module including a light source and a heat source separately.

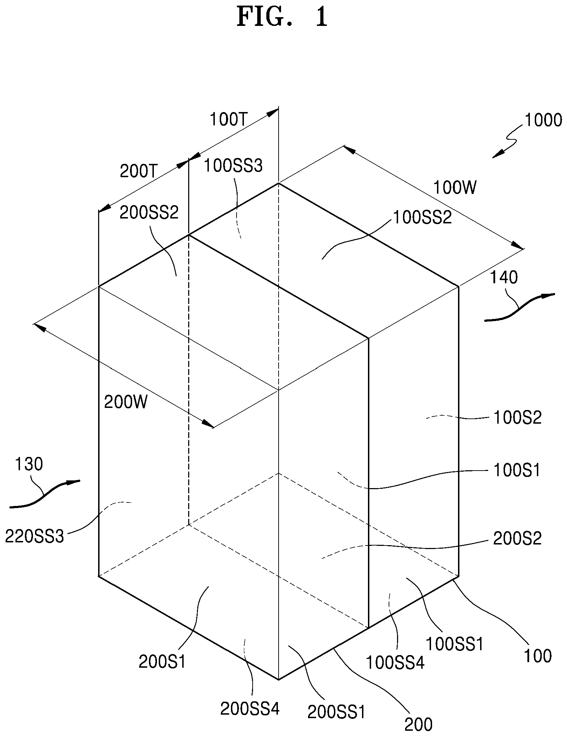

FIG. 1 is a schematic view of an air purification module according to an embodiment.

Referring to FIG. 1, the air purification module 1000 is configured to purify influent unpurified air 130 and discharge the purified air 140, and includes: a catalyst filter 100 including a photocatalyst and an oxidation catalyst; and a light-emitting heat source 200 disposed adjacent to the catalyst filter 100, wherein the light-emitting heat source 200 irradiates, to the catalyst filter 100, light for activating the photocatalyst and provides heat to activate the oxidation catalyst, and a temperature of the light-emitting heat source 200 is higher than a temperature of the unpurified air 130.

The temperature of the light-emitting heat source 200 may be, for example, higher than the temperature of the unpurified air 130 by 20 degrees in Celsius (°C) or more. Since the temperature of the light-emitting heat source 200 is higher than the temperature of the unpurified air 130 flowing into the air purification module 1000 by 20 °C or more, the light-emitting heat source 200 may be able to effectively provide heat to activate the oxidation catalyst. The temperature of the light-emitting heat source 200 may be, for example, higher than the temperature of the unpurified air 130 by 20 °C or more, 40 °C or more, 60 °C or more, 80 °C or more, or 100 °C or more. The higher the temperature of the light-emitting heat source 200 is, the more effectively the oxidation catalyst may be activated. The temperature of the light-emitting heat source 200 may be, for example, about 20 °C to about 1,000 °C, about 50 °C to about 500 °C, about 70 °C to about 300 °C, about 90 °C to about 200 °C, or about 120 °C to about 150 °C. When the temperature of the light-emitting heat source 200 is within the ranges above, the oxidation catalyst may be effectively activated.

The light-emitting heat source 200 may provide heat to activate the oxidation catalyst, and at the same time, may irradiate light for activating the photocatalyst. Here, the light for activating the photocatalyst may be, for example, radio waves, microwaves, ultraviolet rays ("UV"), visible rays, infrared rays ("IR"), X-rays, or a combination thereof. The light irradiated by the light-emitting heat source 200 may be selected according to the type of the photocatalyst. When the catalyst filter 100 includes, for example, a photocatalyst activated by UV, the light-emitting heat source 200 irradiates UV. When the catalyst filter 100 includes, for example, a photocatalyst activated by visible rays, the light-emitting heat source 200 irradiates visible rays. When the catalyst filter 100 includes, for example, a photocatalyst activated by IR, the light-emitting heat source 200 irradiates IR.

The light-emitting heat source 200 may include a first light-emitting heat source surface 200S1 disposed upstream of air flow and a second light-emitting heat source surface 200S2 disposed downstream of air flow. The light-emitting heat source 200 may have a light-emitting heat source thickness 200T from the first light-emitting heat source surface 200S1 to the second light-emitting heat source surface 200S2. The light-emitting heat source 200 may have a light-emitting heat source width 200W defined by a distance between opposite outer peripheral ends of the first light-emitting heat source surface 200S1. The light-emitting heat source width 200W may be, for example, a distance between two side surfaces that are connected to the outer periphery of the first light-emitting heat source surface 200S1 and face each other.

The shape of each of the first light-emitting heat source surface 200S1 and the second light-emitting heat source surface 200S2 may be, for example, circular, oval, trigonal, tetragonal, pentagonal, hexagonal, or octagonal, but is not necessarily limited thereto. Any form suitable for the standard in the art may be used.

Accordingly, the volume and shape of the light-emitting heat source 200 may be defined by, for example, the shape of the first light-emitting heat source surface 200S1, the shape of the second light-emitting heat source surface 200S2, and the light-emitting heat source thickness 200T.

In an embodiment, the light-emitting heat source thickness 200T may be smaller than the light-emitting heat source width 200W. The light-emitting heat source thickness 200T may be, for example, less than or equal to 50 %, less than or equal to 40 %, less than or equal to 30 %, less than or equal to 20 %, less than or equal to 10 %, or less than or equal to 5 % of the light-emitting heat source width 200W. The light-emitting heat source thickness 200T may be, for example, about 0.1 % to about 50 %, about 0.1 % to about 40 %, about 0.5 % to about 30 %, about 0.5 % to about 20 %, about 1 % to about 10 %, or about 1 % to about 5 %, of the light-emitting heat source width 200W. The light-emitting heat source thickness 200T may be, for example, about 1 millimeter (mm) to about 200 mm, about 5 mm to about 150 mm, about 10 mm to about 100 mm, or about 10 mm to about 50 mm. When the light-emitting heat source 200 has a thickness within these ranges, the air purification module 1000 that includes the light-emitting heat source 200 may have a more simplified structure, a further reduced volume, and further improved energy efficiency. When the light-emitting heat source 200 is too thin, the light-emitting heat source 200 may not act as a heat source. When the light-emitting heat source 200 is too thick, the volume of the air purification module 1000 may be increased.

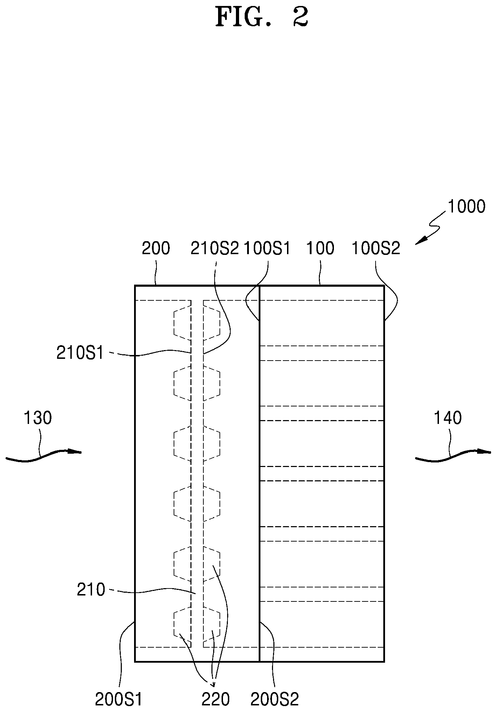

FIG. 2 is a schematic side view of an air purification module according to another embodiment.

Referring to FIG. 2, the light-emitting heat source 200 includes, for example, a support 210 and at least one source 220 for the light-emitting heat supply disposed on one surface or opposite surfaces of the support 210. The light-emitting heat source 200 may include, for example, a plurality of sources 220 for the light-emitting heat supply. The source 220 for the light-emitting heat supply may be, for example, a light-emitting heat source device. The light-emitting heat source device may be, for example, a light-emitting heat source chip. The plurality of sources 220 on one surface 210S1 of the support 210 and the other surface 210S2 facing the one surface 210S1 of the support 210 may be, for example, spaced apart from each other at regular intervals. On the one surface 210S1 of the support 210, the area occupied by the sources 220 for the light-emitting heat supply may be, for example, greater than or equal to 10 %, greater than or equal to 20 %, greater than or equal to 30 %, greater than or equal to 40 %, greater than or equal to 50 %, greater than or equal to 60 %, greater than or equal to 70 %, or greater than or equal to 80 %, of the total area of the one surface 210S1 of the support 210. On the one surface 210S1 of the support 210, the area occupied by the sources 220 for the light-emitting heat supply may be, for example, about 1 % to about 99 %, about 10 % to about 90 %, about 20 % to about 90 %, about 30 % to about 90 %, about 40 % to about 90 %, about 50 % to about 90 %, about 60 % to about 90 %, about 70 % to about 90 %, or about 80 % to about 90 %, of the total area of the one surface 210S1 of the support 210. The support 210 may be, for example, transparent or opaque.

The source 220 for the light-emitting heat supply may, for example, emit light in a first direction protruding from the support 210. In an embodiment, the first direction may be a direction crossing a major surface of the support 210. For example, the first direction may be a direction perpendicular to the major surface of the support 210. The source 220 for the light-emitting heat supply may, for example, emit light in a first direction and a second direction different from the first direction. The second direction may be, for example, a direction opposite to the first direction. The source 220 for the light-emitting heat supply may, for example, emit light in a direction protruding from the support 210. The source 220 for the light-emitting heat supply may, for example, release heat in a direction protruding from the support 210, and at the same time, may release heat in a direction toward the support 210. Although the source 220 for the light-emitting heat supply may, for example, be disposed on one surface of the support 210, the source 220 for the light-emitting heat supply may release heat in all directions. When the source 220 for the light-emitting heat supply is, for example, disposed on opposite surfaces of the support 210, the source 220 for the light-emitting heat supply may emit light to opposite surfaces of the support 210. The support 210 may be, for example, a heat conductor.

A light reflective layer (not shown) may be additionally disposed on the inner side surface of the light-emitting heat source 200 and/or on a portion or all of the surface of the support 210. When the inner side surface of the light-emitting heat source 200 and/or the portion or all of the surface of the support 210 are coated by the light reflective layer, the utilization efficiency of light emitted from the light-emitting heat source 200 may be improved.

A heat insulating layer (not shown) may be additionally disposed on the inner side surface of the light-emitting heat source 200 and/or on a portion or all of the surface of the support 210. When the inner side surface of the light-emitting heat source 200 and/or the portion or all of the surface of the support21 0 are coated by the heat insulating layer, the utilization efficiency of heat released from the light-emitting heat source 200 may be improved.

A light-reflective heat insulation layer (not shown) may be additionally disposed on the inner side surface of the light-emitting heat source 200 and/or on a portion or all of the surface of the support 210. When the inner side surface of the light-emitting heat source 200 and/or the portion or all of the surface of the support210 are coated by the light-reflective heat insulation layer, the utilization efficiency of light and heat from the light-emitting heat source 200 may be improved at the same time. The light-reflective heat insulation layer may have, for example, a light reflective layer/heat insulating layer structure. The light reflective layer may be an aluminum layer, a copper layer, or the like. The heat insulating layer may be a porous polymer layer, a porous glass fiber layer, or the like.

Referring to FIGS. 1 and 2, the air purification module 1000 may be, for example, free of other energy sources that are disposed adjacent to the catalyst filter 100 and different from the light-emitting heat source 200. The air purification module 1000 may not include, for example, other energy sources different from the light-emitting heat source 200, in addition to the light-emitting heat source 200. When the air purification module 1000 does not additionally include other energy sources, the air purification module 1000 may have a simplified structure, a reduced volume, and improved energy efficiency. The other energy sources may be, for example, a light source, a heat source, or a combination thereof. The other energy sources different from the light-emitting heat source 200 may be, for example, a heat source that releases heat to activate the oxidation catalyst. A heat source that releases heat to activate the oxidation catalyst and unintentionally emits light does not correspond to the light-emitting heat source 200 that irradiates light to activate the photocatalyst and releases heat to activate the oxidation catalyst. As the other energy sources, a heat source may be, for example, a heating wire. The other energy sources different from the light-emitting heat source 200 may be, for example, a light source that irradiates light for activating the photocatalyst. A light source that emits light for activating the photocatalyst and unintentionally releases heat does not correspond to the light-emitting heat source 200 that irradiates light to activate the photocatalyst and releases heat to activate the oxidation catalyst. As the other energy sources, the light source may be, for example, a UV source or a visible light source.

Referring to FIGS. 1 and 2, in the air purification module 1000, a heat insulating material (not shown) may be additionally disposed on a portion of all of side surfaces of the light-emitting heat source 200SS1, 200SS2, 200SS3, and 200SS4 and/or side surfaces of the catalyst filter 100SS1, 100SS2, 100SS3, and 100SS4. When the portion or all of the side surfaces of the light-emitting heat source 200SS1, 200SS2, 200SS3, and 200SS4 and/or the side surfaces of the catalyst filter 100SS1, 100SS2, 100SS3, and 100SS4 are coated with the heat insulating material, heat released from the light-emitting heat source 200 may be further effectively transferred to the catalyst filter 100. Here, the heat insulating material may be, for example, a porous foam. The porous foam may be, for example, styrofoam, phenolic foam, nonwoven fabric, aerogel, extruded polystyrene ("XPS"), glass wool, or the like.

Referring to FIGS. 1 and 2, in the air purification module 1000, a light reflective material (not shown) may be additionally disposed on a portion of all of the side surfaces of the light-emitting heat source 200SS, 200SS2, 200SS3, and 200SS4 and/or the side surfaces of the catalyst filter 100SS1, 100SS2, 100SS3, and 100SS4. When the portion or all of the side surfaces of the light-emitting heat source 200SS1, 200SS2, 200SS3, and 200SS4 and/or the side surfaces of the catalyst filter 100SS1, 100SS2, 100SS3, and 100SS4 are coated with the light reflective material, heat released from the light-emitting heat source 200 may be further effectively transferred to the catalyst filter 100. Here, the light reflective material may be, for example, a metal foil. The metal foil may be, for example, an aluminum foil, a copper foil, or the like.

Referring to FIGS. 1 and 2, in the air purification module 1000, a light-reflective insulation material (not shown) may be additionally disposed on a portion of all of the side surfaces of the light-emitting heat source 200SS, 200SS2, 200SS3, and 200SS4 and/or the side surfaces of the catalyst filter 100SS1, 100SS2, 100SS3, and 100SS4. When the portion or all of the side surfaces of the light-emitting heat source 200SS1, 200SS2, 200SS3, and 200SS4 and/or the side surfaces of the catalyst filter 100SS1, 100SS2, 100SS3, and 100SS4 are coated with the light-reflective insulation material, light and heat from the light-emitting heat source 200 may be further effectively transferred to the catalyst filter 100. Here, the light-reflective insulation material may be, for example, a multilayer structure in which a light reflective layer and a heat insulating layer are stacked. Here, the light reflective layer may be, for example, a metal layer. The metal layer may be, for example, aluminum, copper, or an alloy thereof. The heat insulating layer may be, for example, a porous polymer layer, a porous glass fiber layer, or the like. The light-reflective insulation material may have, for example, a structure consisting of 2 layers to 100 layers. The light-reflective insulation material may have, for example, a light reflective layer/heat insulating layer structure or a light reflective layer/heat insulating layer/light reflective layer structure.

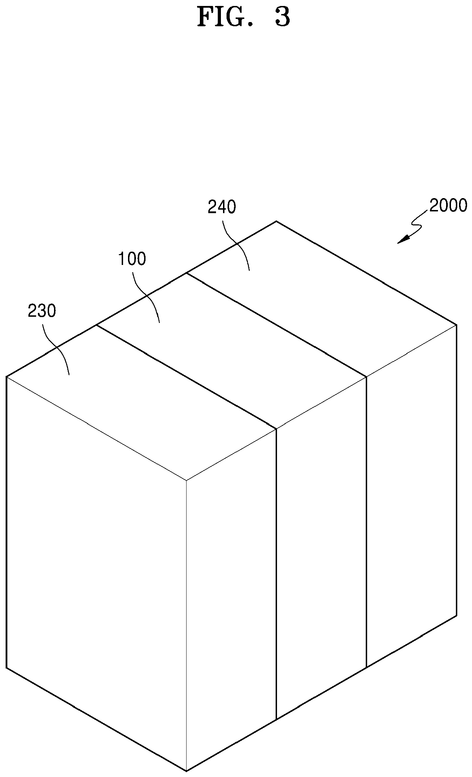

FIG. 3 is a schematic view of an air purification module including other energy sources different from a light-emitting heat source.

Referring to FIG. 3, other energy sources included in an air purification module 2000 may be, for example, a heat source and/or a light source. The catalyst filter 100 may include a photocatalyst and an oxidation catalyst. To activate both the photocatalyst and the oxidation catalyst, the air purification module 2000 includes a heat source 230 for emitting heat to activate the oxidation catalyst and a light source 240 for irradiating light for activating the photocatalyst, separately. The air purification module 2000 may separately include a plurality of energy sources.

Referring to FIGS. 1 and 3, a first volume of the air purification module 1000 that does not include other energy sources may be, for example, smaller than a second volume of the air purification module 2000 that includes other energy sources. When the first volume of the air purification module 1000 that does not include other energy sources is smaller than the second volume of the air purification module 2000 that includes other energy sources, the air purification module 1000 may have a more rigid structure, and may be able to implement the miniaturization of the air purification module 1000. The first volume may be, for example less than or equal to 80 % less than or equal to 70 %, less than or equal to 60 %, less than or equal to 50 %, less than or equal to 45 %, or less than or equal to or 40 %, of the second volume. The first volume may be, for example, about 10 % to about 80 %, 10 % to about 70 %, 10 % to about 60 %, 10 % to about 50 %, 10 % to about 45 %, or 10 % to about 40 % of the second volume.

A first power consumption of the air purification module 1000 that does not include other energy sources may be, for example, smaller than a second power consumption of the air purification module 2000 that includes other energy sources. When the first power consumption of the air purification module 1000 that does not include other energy sources is smaller than the second power consumption of the air purification module 2000 that includes other energy sources, the air purification module 1000 may be able to exhibit improved energy efficiency. The first power consumption may be, for example less than or equal to 80 % less than or equal to 70 %, less than or equal to 60 %, less than or equal to 50 %, less than or equal to 45 %, or less than or equal to or 40 %, of the second power consumption. The first power consumption may be, for example, about 10 % to about 80 %, 10 % to about 70 %, 10 % to about 60 %, 10 % to about 50 %, 10 % to about 45 %, or 10 % to about 40 % of the second power consumption.

The catalyst filter 100 may include a first catalyst filter surface 100S1 disposed upstream of air flow and a second catalyst filter surface 100S2 disposed downstream of air flow. The catalyst filter 100 may have, for example, a catalyst filter thickness 100T from the first catalyst filter surface 100S1 to the second catalyst filter surface 100S2. The direction used to measure the thickness 100T is defined as a thickness direction of the catalyst filter 100. The catalyst filter 100 may have, for example, a catalyst filter width 100W defined by a distance between opposite outer peripheral ends of the first catalyst filter surface 100S1. The catalyst filter width 100W may be, for example, a distance between two side surfaces that are connected to the outer periphery of the first catalyst filter surface 100S1 and face each other.

The shape of each of the first catalyst filter surface 100S1 and the second catalyst filter surface 100S2 may be, for example, circular, oval, trigonal, tetragonal, pentagonal, hexagonal, or octagonal, but is not necessarily limited thereto. Any form suitable for the standard in the art may be used.

Accordingly, the volume and shape of the catalyst filter 100 may be defined by, for example, the shape of the first catalyst filter surface 100S1, the shape of the second catalyst filter surface 100S2, and the catalyst filter thickness 100T.

In an embodiment, the catalyst filter thickness 100T may be smaller than the catalyst filter width 100W. The catalyst filter thickness 100T may be, for example, less than or equal to 50 %, less than or equal to 40 %, less than or equal to 30 %, less than or equal to 20 %, less than or equal to 10 %, or less than or equal to 5 % of the catalyst filter width 100W. The catalyst filter thickness 100T may be, for example, about 0.1 % to about 50 %, about 0.1 % to about 40 %, about 0.5 % to about 30 %, about 0.5 % to about 20 %, about 1 % to about 10 %, or about 1 % to about 5 %, of the catalyst filter width 100W. The thickness 100T of the catalyst filter 100 may be, for example, about 1 mm to about 200 mm, about 5 mm to about 150 mm, about 10 mm to about 100 mm, or about 10 mm to about 50 mm. When the catalyst filter 100 has a thickness within these ranges, the air purification module 1000 that includes the catalyst filter 100 may have a more simplified structure, a further reduced volume, and further improved energy efficiency. When the catalyst filter 100 is too thin, the amount of a catalyst included in the catalyst filter 100 may be excessively reduced, and thus the air may not be effectively purified. When the catalyst filter 100 is too thick, light emitted from the light-emitting heat source 200 may not be irradiated to the entire catalyst filter 100, or heat released from the light-emitting heat source 200 may not be transferred to the entire catalyst filter 100.

A light reflective layer (not shown) may be additionally disposed on a portion or all of the inner surface of the catalyst filter 100. When the portion or all of the inner surface of the catalyst filter 100 is coated with the light reflective layer, the utilization efficiency of light emitted from the light-emitting heat source 200 may be improved.

A heat insulating layer (not shown) may be additionally disposed on a portion or all of the inner surface of the catalyst filter 100. When the portion or all of the inner surface of the catalyst filter 100 is coated with the heat insulating layer, the utilization efficiency of heat released from the light-emitting heat source 200 may be improved.

A light-reflective heat insulation layer (not shown) may be additionally disposed on a portion or all of the inner surface of the catalyst filter 100. When the portion or all of the inner surface of the catalyst filter 100 is coated with the light-reflective heat insulation layer, the utilization efficiency of light and heat from the light-emitting heat source 200 may be improved at the same time. The light-reflective heat insulation layer may have, for example, a light reflective layer/heat insulating layer structure. The light reflective layer may be an aluminum layer, a copper layer, or the like. The heat insulating layer may be a porous polymer layer, a porous glass fiber layer, or the like.

The catalyst filter 100 may include the photocatalyst and the oxidation catalyst, and the photocatalyst and the oxidation catalyst may be disposed on a light reflective layer, a heat insulating layer, and/or a light-reflective heat insulation layer.

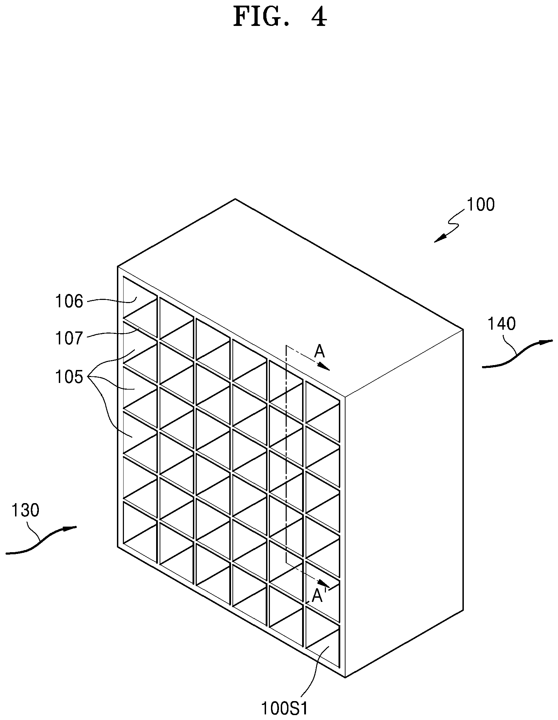

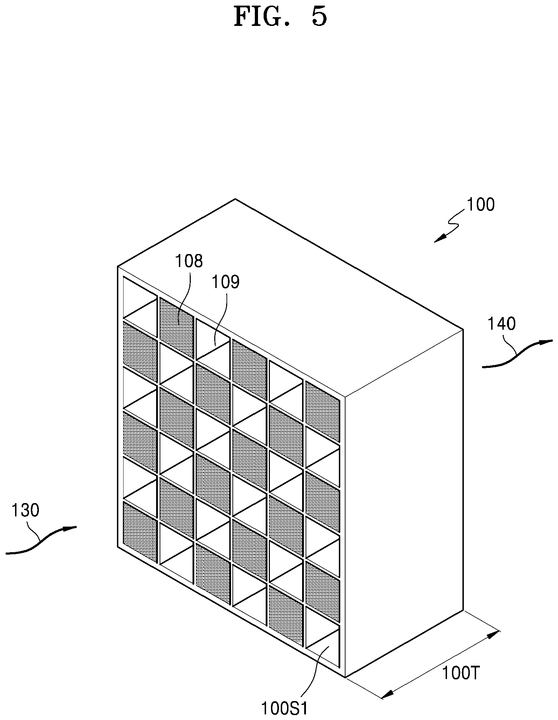

FIG. 4 is a schematic view of the catalyst filter according to an embodiment. FIG. 5 is a schematic view of the catalyst filter according to another embodiment.

Referring to FIGS. 4 and 5, the catalyst filter 100 may include, for example, a plurality of cells 105 that form a path of air flow along the catalyst filter thickness 100T direction on the first catalyst filter surface 100S1 and are disposed parallel adjacent to each other.

The plurality of cells 105 disposed parallel may be, for example, disposed regularly or irregularly. The plurality of cells 105 disposed parallel may be, for example, disposed periodically or non-periodically.

When the catalyst filter 100 includes the plurality of cells 105 disposed with such a pattern, pollutants may be further effectively removed from the unpurified air 130.

Referring to FIG. 4, the catalyst filter 100 includes a plurality of cells 105 that are disposed parallel adjacent to each other, and the plurality of cells 105 include through-holes 106 and partition walls 107 defining the shape of the through-holes 106.

The through-holes 106 may form a path of air flow by passing through the first catalyst filter surface 100S1 and the second catalyst filter surface 100S2. The partition walls 107 may be disposed between adjacent through-holes 106.

The area occupied by the through-holes 106 in the first catalyst filter surface 100S1 may be, for example, about 50 % to about 99 %, about 60 % to about 99 %, about 70 % to about 99%, about 80 % to about 99%, or about 90 % to about 99 %, of the total area of the first catalyst filter surface 100S1.

Referring to FIG. 5, the catalyst filter 100 may include a plurality of cells 105 disposed parallel adjacent to each other, and the plurality of cells 105 may include a protrusion 108 and/or a depression 109.

When the plurality of cells 105 include the protrusion 108 and/or the depression 109, the area where the unpurified air 130 contacts the catalyst filter 100 may be maximized. The protrusion 108 and/or the depression 109 may be, for example, porous. When the protrusion 108 and/or the depression 109 is porous, the unpurified air 130 may be purified by passing through the protrusion 108 and/or the depression 109.

An inlet of the through-hole 106 may be, for example, circular, oval, trigonal, tetragonal, pentagonal, hexagonal, octagonal, or a combination thereof in a view from the thickness direction of the catalyst filler 100, but is not limited thereto. Any shape suitable for purifying the unpurified air 130 may be used. A surface of the protrusion 108 may be, for example, circular, oval, trigonal, tetragonal, pentagonal, hexagonal, octagonal, or a combination thereof, but is not limited thereto. Any shape suitable for purifying the unpurified air 130 may be used. A bottom portion of the depression 109 may be, for example, circular, oval, trigonal, tetragonal, pentagonal, hexagonal, octagonal, or a combination thereof in a view from the thickness direction of the catalyst filler 100, but is not limited thereto. Any shape suitable for purifying the unpurified air 130 may be used.

A diameter of the through-hole 106 may, for example, increase, decrease, or remain constant along the catalyst filter thickness 100T direction, but is not limited thereto. Any pattern suitable for purifying the unpurified air 130 may be used. A diameter of the protrusion 108 may, for example, increase, decrease, or remain constant along the catalyst filter thickness 100T direction, but is not limited thereto. Any pattern suitable for purifying the unpurified air 130 may be used. A diameter of the depression 109 may, for example, increase, decrease, or remain constant along the catalyst filter thickness 100T direction, but is not limited thereto. Any pattern suitable for purifying the unpurified air 130 may be used.

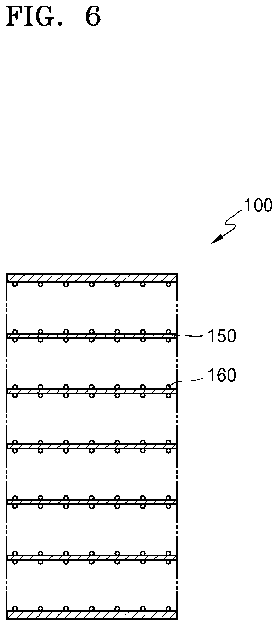

FIG. 6 is a cross-sectional view taken along lines A and A' of the catalyst filter 100 of FIG. 4.

The catalyst filter 100 may include, for example, a solid substrate 150 and a catalyst 160 coated on the solid substrate 150. The catalyst 160 may include a photocatalyst and an oxidation catalyst.

The photocatalyst may be a catalyst activated by light emitted from the light-emitting heat source 200. The oxidation catalyst may be a catalyst activated by heat released from the light-emitting heat source 200.

The photocatalyst may include, for example, a first metal, a first metal oxide, a first metal carbide, a first metal nitride, a first metal oxynitride, or a combination thereof.

The first metal may include, for example, Ti, Zn, Zr, Ta, Nb, W, an alloy thereof, or a combination thereof, but is not limited thereto. Any metal available as the photocatalyst in the art may be used.

The first metal oxide may be, for example, TiOx (where 0 <x≤2), ZnOx (where 0<x≤2), ZrOx (where 0<x≤2), TaOx (where 0<x≤2), NbxOy (where 0<x≤2, 0<y≤5), WOx (where 0<x≤3), or a combination thereof.

The first metal carbide may be, for example, TixCy (where 0 <x≤3, 0<y≤2), ZnCx (where 0<x≤2), ZrCx (where 0<x≤2), TaCx (where 0<x≤2), NbCx (where 0<x≤2), WCx (where 0<x≤2), or a combination thereof.

The first metal nitride may be, for example, TiNₓ (where 0<x≤1), ZnNₓ (where 0<x≤1), ZrNₓ (where 0<x≤1), TaNₓ (where 0<x≤1), NbₓN_{y} (where 0<x≤1), WNₓ (where 0<x≤2), or a combination thereof.

The first metal oxynitride may be, for example, TiNₓO_{y} (where 0<x≤2 and 0<y≤2), ZnNₓO_{y} (where 0<x≤2 and 0<y≤2), ZrNₓO_{y} (where 0<x≤2 and 0<y≤2), TaNₓO_{y} (where 0<x≤2 and 0<y≤2), NbNₓO_{y} (where 0<x≤3 and 0<y≤3), WNₓO_{y} (where 0<x≤3 and 0<y≤2), or a combination thereof.

The photocatalyst may be in the form of a particle, and the photocatalyst particle may have, for example, a spherical shape, a tube shape, a rod shape, a fiber shape, a sheet shape, or a combination thereof.

The photocatalytic particle may be, for example, a primary particle or a secondary particle which is a combination of a plurality of primary particles.

The oxidation catalyst may include, for example, a second metal, a second metal oxide, a second metal carbide, a second metal nitride, a second metal oxynitride, or a combination thereof.

The second metal may be different from the first metal. The second metal may include, for example, Ti, Mn, Co, Ce, Al, Fe, Ni, Na, In, Bi, Sn, Pt, Au, Ag, Zn, Pd, an alloy thereof, or a combination thereof, but is not limited thereto. Any metal available as the oxidation catalyst in the art may be used.

The second metal oxide may include, for example, TiOₓ (where 0<x≤2), MnOₓ (where 0<x≤2), CoₓO_{y}(where 0<x≤3 and 0<y≤4), CeOₓ(where 0<x≤2), MnOₓ-TiO_{y}(where 0<x≤2 and 0<y≤2), AlₓO_{y}(where 0<x≤2 and 0<y≤3), FeₓO_{y}(where 0<x≤2 and 0<y≤3), NiOₓ (where 0<x≤1), MnOₓ-Mn_{y}O_{z} (where 0<x≤2, 0<y≤3, and 0<z≤4), NaInOₓ (where 0<x≤2), BiₓWO_{y} (where 0<x≤2 and 0<y≤6), SnOₓ (where 0<x≤2), or a combination thereof. The second metal oxide may include, for example, TiO₂, MnO₂, Co₃O₄, CeO₂, MnO₂-TiO₂, Al₂O₃, Fe₂O₃, NiO, MnO₂-Mn₃O₄, NaInO₂, Bi₂WO₆, SnO₂, or a combination thereof.

The second metal carbide may include, for example, titanium carbide, manganese carbide, cobalt carbide, cerium carbide, aluminum carbide, iron carbide, nickel carbide, or a combination thereof.

The second metal nitride may include, for example, titanium nitride, manganese nitride, cobalt nitride, cerium nitride, aluminum nitride, iron nitride, nickel nitride, or a combination thereof.

The second metal oxynitride may include, for example, titanium oxynitride, manganese oxynitride, cobalt oxynitride, cerium oxynitride, aluminum oxynitride, iron oxynitride, nickel oxynitride, or a combination thereof.

The oxidation catalyst may be in the form of a particle, and the oxidation catalyst particle may have, for example, a spherical shape, a tube shape, a rod shape, a fiber shape, a sheet shape, or a combination thereof.

The oxidation catalyst particle may be, for example, a primary particle or a secondary particle which is a combination of a plurality of primary particles.

The solid substrate 150 may be, for example, a porous substrate or a non-porous substrate. The porous substrate may be a substrate including a plurality of pores through which the air can penetrate. A size of the pores included in the porous substrate may be, for example, about 0.1 nanometers (nm) to about 100 nm, about 0.1 nm to about 50 nm, about 0.1 nm to about 10 nm, or about 1 nm to about 5 nm. The non-porous substrate may be a substrate that the air cannot penetrate. The non-porous substrate may be, for example, a substrate that does not include pores.

The solid substrate 150 may include, for example, ceramic, carbon, polymer, metal, nonwoven fabric, woven fabric, or a combination thereof.

The catalyst filter 100 may be mounted on various indoor and outdoor air purification apparatuses, such as air purifiers, air purification facilities, air conditioning facilities, and the like, to remove gas of Volatile Organic Compound ("VOC") or the like or fine dust from the unpurified air 130. The catalyst filter 100 may also remove, in addition to VOC, nitrogen oxides (NOx), sulfur oxides (SOx), ammonia (NH₃), odor substances, germs, pathogens, bacteria, and the like.

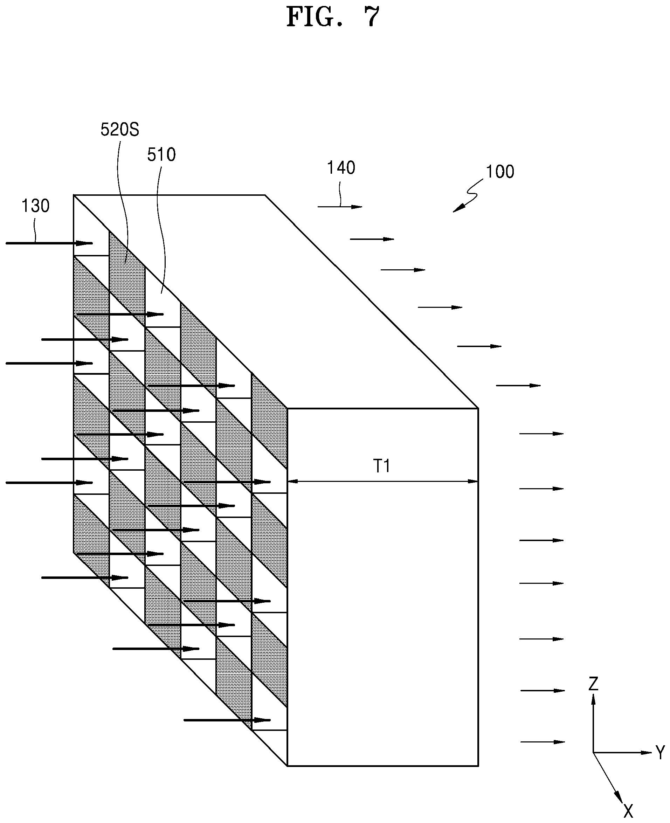

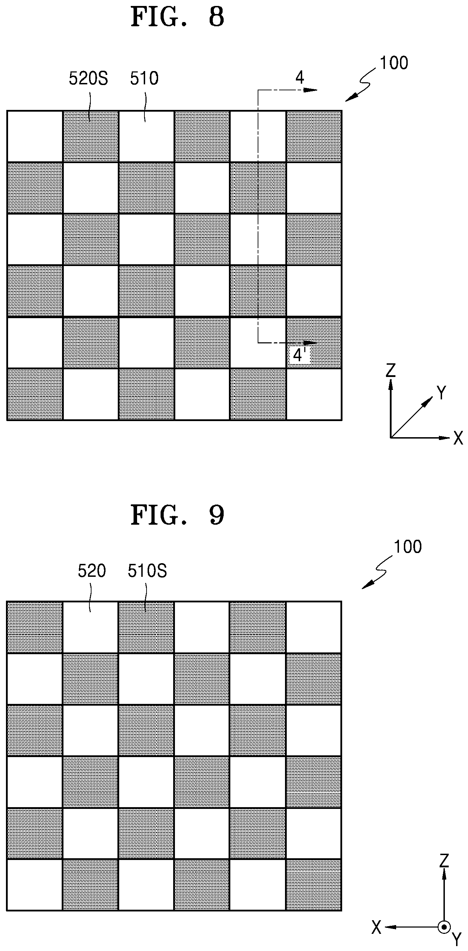

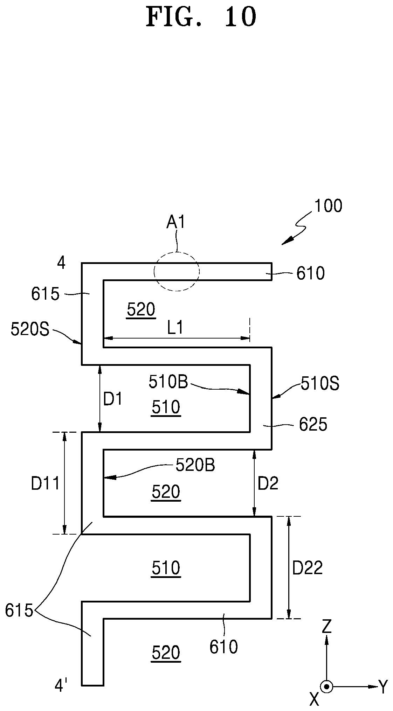

FIG. 7 is a schematic view of the catalyst filter 100 according to an embodiment. FIG. 8 is a front view of the influent surface of the catalyst filter 100 of FIG. 7 where the unpurified air 130 flows in. FIG. 9 is a front view of the exhaust surface of the catalyst filter 100 of FIG. 7 where the purified air 140 flows out. FIG. 10 is a cross-sectional view taken along 4-4' direction of the catalyst filter 100 of FIG. 8.

Referring to FIG. 7, the catalyst filter 100 includes an influent surface where the unpurified air 130 flows in and an effluent surface where the purified air 140 flows out. The unpurified air 130 may include, for example, a material decomposable by a photocatalyst and a material decomposable by an oxidation catalyst.

The unpurified air 130 may include a particulate compound and a gaseous compound. The catalyst filter 100 may have a thickness T1 defined by a direction (Y-axis direction) from the influent surface toward the effluent surface.

The catalyst filter 100 may include a plurality of first depressions 510 having an inlet adjacent to the influent surface where the unpurified air 130 flows in a bottom adjacent to the effluent surface where the purified air 140 flows out. The unpurified air 130 may flow into the catalyst filter 100 through a plurality of first depressions 510. The plurality of first depressions 510 may be arranged regularly and/or periodically. The plurality of first depressions 510 may be arranged parallel to each other.

The catalyst filter 100 may include a plurality of first surfaces 520S exposed to the influent surface where the unpurified air 130 flows in. The first surface 520S may correspond to, for example, a surface of a first protrusion. The plurality of first surfaces 520S may be arranged regularly. The plurality of first surfaces 520S may be disposed between the plurality of first depressions 510.

The plurality of first surfaces 520S may be spaced apart between the plurality of first depressions 510 spaced apart in one direction along the surface of the influent surface, e.g., along the X-axis direction and/or the Z-axis direction. The plurality of first depressions 510 and the plurality of first surfaces 520S may be alternately disposed in one direction along the surface of the influent surface, e.g., along the X-axis direction and/or the Z-axis direction. Accordingly, one first depression 510 may be surrounded by four first surfaces 520S, and one first surface 520S may be surrounded by four first depressions 510.

FIG. 8 is a front view of the influent surface of the catalyst filter 100 of FIG. 7 where the unpurified air 130 flows in. FIG. 9 is a front view of the effluent surface of the catalyst filter 100 of FIG. 7 where the purified air 140 flows out.

Referring to FIG. 8, the influent surface of the catalyst filter 100 may include a plurality of first depressions 510 and a plurality of first surfaces 520S.

Referring to FIG. 9, the effluent surface of the catalyst filter 100 may include a plurality of second depressions 520 and a plurality of second surfaces 510S.

The plurality of second depressions 520 may be outlets through which the purified air 140 is discharged. The purified air 140 discharged through the second depression 520 may be an air without pollutants which are removed from the unpurified air 130 influent while passing through the first depression 510, or may be an air containing a harmless gas which are obtained by decomposition of pollutants.

The plurality of second depressions 520 may be arranged regularly and/or periodically. The plurality of second surfaces 510S may be arranged regularly. The plurality of second surfaces 510S may be disposed between the plurality of second depressions 520. The second surface 510S may correspond to, for example, a surface of a second protrusion.

The plurality of second surfaces 510S may correspond to the plurality of first depressions 510, and the plurality of second depressions 520 may correspond to the plurality of first surfaces 520S.

Referring to FIGS. 8 and 9, the second surface 510S may correspond to the bottom portion of the first depression 510, and the first surface 520S may correspond to the bottom portion of the second depression 520.

FIG. 10 is a cut section taken along 4-4' direction of the catalyst filter 100 of FIG. 8.

The catalyst filter 100 may have a single body structure or a single body frame. The catalyst filter 100 may have a frame formed entirely of the same material, for example, a ceramic material, a polymer material, a metal material, or the like. The catalyst filter 100 may have a structure integrated as a whole, such as a single body or a monolithic structure. Alternatively, the catalyst filter 100 may be a multilayer structure or a multilayer frame. The catalyst filter 100 may have, for example, a multilayer structure including a solid substrate and a photocatalyst and an oxidation catalyst disposed on the solid substrate, although not shown in the drawing.

Referring to FIG. 10, the catalyst filter 100 may be a structure having a frame in which a plurality of first depressions 510 and a plurality of second depressions 520 are sequentially disposed in the Z-axis direction. The catalyst filter 100 may include a plurality of horizontal regions 610 and a plurality of vertical regions 615 and 625. The plurality of horizontal regions 610 may be spaced apart from each other along the Z-axis direction. The Z-axis direction may correspond to the vertical direction. The plurality of horizontal regions 610 may be disposed parallel to each other along the Y-axis direction. A length of each of the plurality of horizontal regions 610 may be equal to or different from each other. The plurality of horizontal regions 610 may be disposed between the plurality of vertical regions 615 and 625. The plurality of horizontal regions 610 may be physically connected to each other through the plurality of vertical regions 615 and 625. The plurality of vertical regions 615 and 625 may be disposed parallel to each other, and may be spaced apart from each other. The plurality of vertical regions 615 and 625 may be spaced apart from each other along the Z-axis direction. The Z-axis direction may correspond to the vertical direction. The plurality of vertical regions 615 and 625 may be disposed parallel to each other along the Y-axis direction. A length of each of the plurality of vertical regions 615 and 625 may be equal to or different from each other. The plurality of vertical regions 615 and 625 may be disposed between the plurality of horizontal regions 610. The plurality of vertical regions 615 and 625 may be physically connected to teach other through the plurality of horizontal regions 610. The plurality of vertical regions 615 and 625 may include a plurality of first vertical regions 615 and a plurality of second vertical regions 625. The plurality of first vertical regions 615 and the plurality of second vertical regions 625 may be spaced apart from each other along the Y-axis direction. The plurality of first vertical regions 615 may be spaced apart from each other along the Z-axis direction. The plurality of second vertical regions 625 may be spaced apart from each other in the Z-axis direction. The plurality of first vertical regions 615 may be disposed on the influent surface to which the unpurified air 130 is supplied. The plurality of second vertical regions 625 may be disposed on the effluent surface from which the purified air 140 is discharged.

The plurality of horizontal regions 610 may correspond to walls of the first depression 510 and the second depression 520. The plurality of horizontal regions 610 may each be positioned between the first depression 510 and the second depression 520 to form boundaries between respective depressions 510 and 520. The walls may correspond to side walls of the first depression 510 and the second depression 520. A thickness of each of the plurality of horizontal regions 610 may be equal to or different from each other. The thicknesses of the plurality of horizontal regions 610 may be equal to or different from the thicknesses of the plurality of vertical regions 615 and 625. The horizontal region 610 as a wall of the first depression 510 may be spaced apart from each other by a first distance D1 in the Z-axis direction. The horizontal region 610 as a wall of the second depression 520 may be spaced apart from each other by a second distance D2 in the Z-axis direction. The first distance D1 and the second distance D2 may be equal to or different from each other. The diameters and/or areas of the inlets of the first depression 510 and the second depression 520 may be equal to or different from each other. The lengths L1 in the Y-axis direction of each of the plurality of horizontal regions 610 may be equal to or different from each other. The depths of the first depression 510 and the second depression 520 may be defined by the length L1 in the Y-axis direction of the horizontal region 610. The depths of the first depression 510 and the second depression 520 may be equal to or different from each other. The plurality of first vertical regions 615 may form the bottom portions of the plurality of second depressions 520. The plurality of second vertical regions 625 may form the bottom portions of the plurality of first depressions 510. Here, the air permeability of the bottom portion of the first depression 510 and the bottom portion of the second depression 520 may be the same as or different from each other. The diameter D11 of the first vertical regions 615 and the diameter D22 of the second vertical regions 625 may be equal to or different from each other. The thicknesses in the Y-axis direction of the f first vertical regions 615 and the second vertical regions 625 may be equal to or different from each other.

The plurality of horizontal regions 610 and the plurality of vertical regions 615 and 625 may have a structure integrated as a whole, such as a single body or a monolithic structure, wherein the structure may be formed of the same materials.

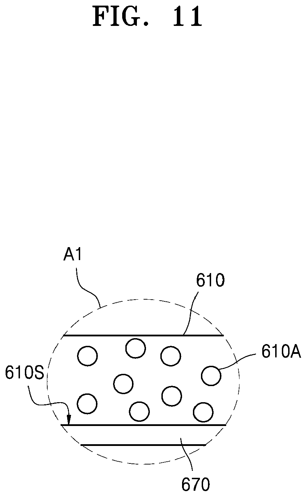

FIG. 11 is an enlarged view of a first area A1 of the horizontal region 610 of FIG. 10.

Referring to FIG. 11, the horizontal region 610 may include pores 610A. The vertical regions 615 and 625 may or may not include pores.

When the horizontal region 610 and the vertical regions 615 and 625 include pores, and the pore density of the vertical regions 615 and 625 may be higher or lower than or the same as a pore density of the horizontal region 610.

For example, the first vertical region 615 may include pores, and the second vertical region 625 may not include pores. Alternatively, the first vertical region 615 may not include pores, and the second vertical region 625 may include pores.

When the first vertical region 615 and the second vertical region 625 include pores, and the pore density of the vertical region 625 may be higher or lower than or the same as a pore density of the first vertical region 615.

A catalyst layer 670 including the above-described photocatalyst for air purification may be disposed on a surface 610S of the horizontal region 610 on which the energy is irradiated. The catalyst layer 670 may include the photocatalyst and the oxidation catalyst. The catalyst layer 670 may be disposed, for example, on both the horizontal region 610 and the vertical regions 615 and 625.

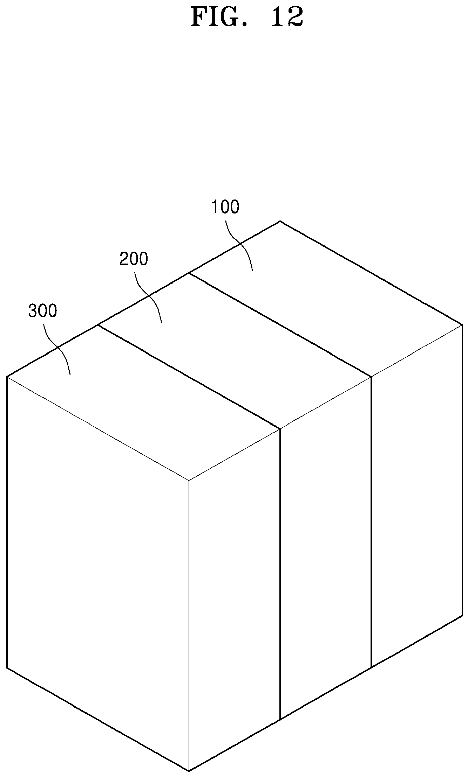

FIG. 12 is a schematic view of an air purification module including a dust collecting filter according to an embodiment.

The air purification module 1000 may further include, for example, a dust collecting filter 300.

The dust collecting filter 300 may be, for example, disposed upstream of air flow, compared to the catalyst filter 100. When the dust collecting filter 300 is disposed upstream of air flow, compared to the catalyst filter 100, the dust collecting filter 300 may first remove particles included in the unpurified air. By first removing dust particles included in the unpurified air by the dust collecting filter 300, the air including the reduced dust particles may be supplied to the catalyst filter 100. Accordingly, the catalyst filter 100 may be able to more effectively purify the air supplied thereto.

The thickness and width of the dust collecting filter 300 may be, for example, the same as those of the catalyst filter 100. The dust collecting filter 300 may include a porous substrate. When the dust collecting filter 300 includes a porous substrate, dust particles included in the unpurified air may be easily adsorbed on the porous substrate. The porous substrate may include, for example, activated carbon.

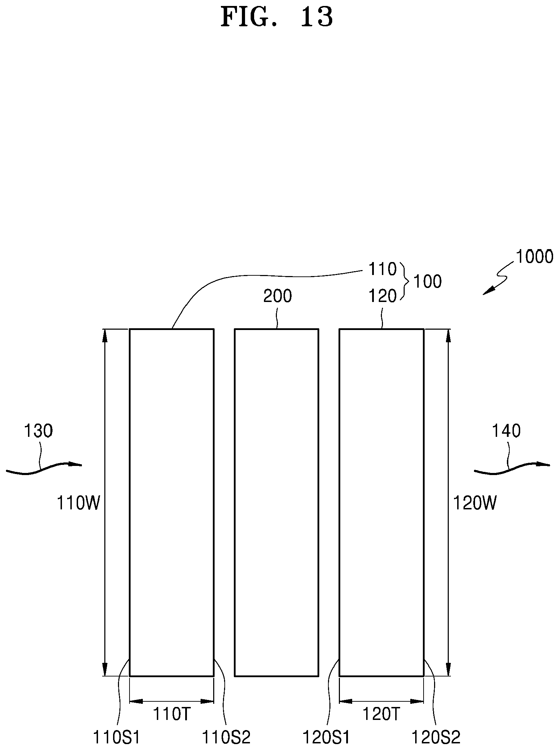

FIG. 13 is a schematic side view of the air purification module according to another embodiment.

Referring to FIG. 13, the air purification module 1000 includes the catalyst filter 100 and the light-emitting heat source 200. The catalyst filter 100 may include: a photocatalyst filter 110 disposed adjacent to the light-emitting heat source 200 and including a photocatalyst; and an oxidation catalyst filter 120 disposed adjacent to the light-emitting heat source 200 and including an oxidation catalyst. When the photocatalyst filter 110 and the oxidation catalyst filter 120 are disposed adjacent to the light-emitting heat source 200, the light-emitting heat source 200 may activate the photocatalyst in the photocatalyst filter 110 and the oxidation catalyst in the oxidation catalyst filter 120 at the same time. Thus, compared to an air purification module including a light source and a heat source separately, the air purification module 1000 may have a simplified structure, a compact volume, and improved energy efficiency.

The photocatalyst filter 110 may be, for example, disposed upstream of air flow, compared to the light-emitting heat source 200. Alternatively, the photocatalyst filter 110 may be, for example, disposed downstream of air flow, compared to the light-emitting heat source 200. The oxidation catalyst filter 120 may be, for example, disposed upstream of air flow, compared to the light-emitting heat source 200. When the oxidation catalyst filter 120 is disposed downstream of air flow, compared to the light-emitting heat source 200, heat released from the light-emitting heat source 200 may be more effectively used. For example, the unpurified air 130 may flow into the photocatalyst filter 110 disposed upstream of air flow, and the purified air 140 may be discharged from the oxidation catalyst filter 120 disposed downstream of air flow.

The photocatalyst filter 110 may be, for example, disposed closer to the light-emitting heat source 200 than to the oxidation catalyst filter 120. When the photocatalyst filter 110 is disposed closer to the light-emitting heat source 200 than to the oxidation catalyst filter 120, the photocatalyst included in the photocatalyst filter 110 may be used more effectively used. The oxidation catalyst filter 120 may be, for example, disposed closer to the light-emitting heat source 200 than to the photocatalyst filter 110. When the oxidation catalyst filter 120 is disposed closer to the light-emitting heat source 200 than to the photocatalyst filter 110, the oxidation catalyst included in the oxidation catalyst filter 120 may be used more effectively used.

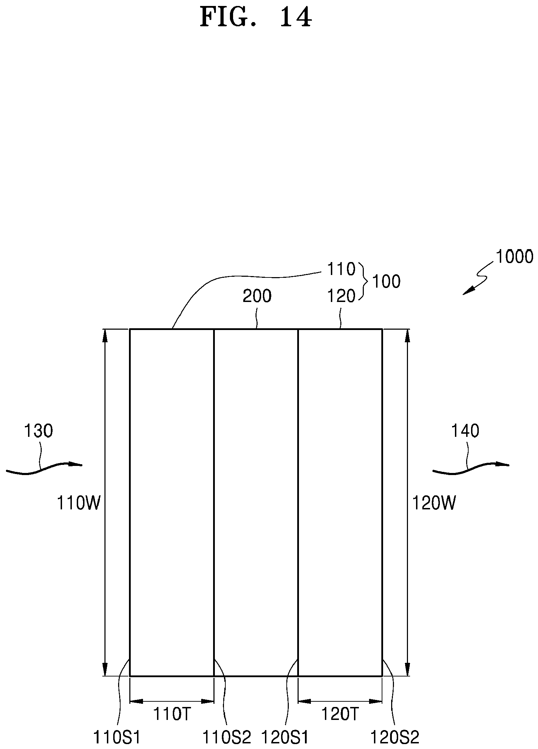

FIG. 14 is a schematic side view of the air purification module according to another embodiment.

Referring to FIG. 14, the air purification module 1000 includes the photocatalyst filter 110 and the oxidation catalyst filter 120. The photocatalyst filter 110 may be disposed upstream of air flow, compared to the light-emitting heat source 200, and accordingly, may be in contact with one surface of the light-emitting heat source 200. The oxidation catalyst filter 120 may be, for example, disposed downstream of air flow, compared to the light-emitting heat source 200, and accordingly, may be in contact with another surface of the light-emitting heat source 200. When the photocatalyst filter 110 and the oxidation catalyst filter 120 may be in contact with the one surface of the light-emitting heat source 200 and the another surface facing the one surface, respectively, the volume of the air purification module 1000 may be further reduced and the structure of the air purification module 1000 may be more simplified.

Referring to FIGS. 13 and 14, the photocatalyst filter 110 includes a first photocatalyst filter surface 110S1 disposed upstream of air flow and a second photocatalyst filter surface 110S2 disposed downstream of air flow. The photocatalyst filter 110 may have, for example, a photocatalyst filter thickness 110T from the first photocatalyst filter surface 110S1 to the second photocatalyst filter surface 110S2. The photocatalyst filter 110 may have, for example, a photocatalyst filter width 110W defined by a distance between opposite outer peripheral ends of the first photocatalyst filter surface 110S1. The photocatalyst filter width 110W may be, for example, a distance between two side surfaces that are connected to the outer periphery of the first photocatalyst filter surface 110S1 and face each other.

The shape of each of the first photocatalyst filter surface 110S1 and the second photocatalyst filter surface 110S2 may be, for example, circular, oval, trigonal, tetragonal, pentagonal, hexagonal, or octagonal, but is not necessarily limited thereto. Any form suitable for the standard in the art may be used.

Accordingly, the volume and shape of the photocatalyst filter 110 may be defined by, for example, the shape of the first photocatalyst filter surface 110S1, the shape of the second photocatalyst filter surface 110S2, and the photocatalyst filter thickness 110T.

In an embodiment, the photocatalyst filter thickness 110T may be smaller than the photocatalyst filter width 110W. The photocatalyst filter thickness 110T may be, for example, less than or equal to 50 %, less than or equal to 40 %, less than or equal to 30 %, less than or equal to 20 %, less than or equal to 10 %, or less than or equal to 5 %, of the photocatalyst filter width 110W. The photocatalyst filter thickness 110T may be, for example, about 0.1 % to about 50 %, about 0.1 % to about 40 %, about 0.5 % to about 30 %, about 0.5 % to about 20 %, about 1 % to about 10 %, or about 1 % to about 5 %, of the photocatalyst filter width 110W. The photocatalyst filter thickness 110T may be, for example, about 0.1 mm to about 100 mm, about 0.5 mm to about 70 mm, about 1 mm to about 50 mm, about 5 mm to about 50 mm, or about 10 mm to about 50 mm. When the photocatalyst filter 110 has a thickness within these ranges, the air purification module 1000 that includes the photocatalyst filter 110 may have a more simplified structure, a further reduced volume, and further improved energy efficiency. When the photocatalyst filter 110 is too thin, the amount of a catalyst included in the photocatalyst filter 110 may be excessively reduced, and thus the air may not be effectively purified. When the photocatalyst filter 110 is too thick, light emitted from the light-emitting heat source 200 may not be irradiated to the entire photocatalyst filter 110.

Referring to FIGS. 13 and 14, the oxidation catalyst filter 120 includes a first oxidation catalyst filter surface 120S1 disposed upstream of air flow and a second oxidation catalyst filter surface 120S2 disposed downstream of air flow. The oxidation catalyst filter 120 may have, for example, an oxidation catalyst thickness 120T from the first oxidation catalyst filter surface 120S1 to the second oxidation catalyst filter surface 120S2. The oxidation catalyst filter 120 may have, for example, an oxidation catalyst filter width 120W defined by a distance between opposite outer peripheral ends of the first oxidation catalyst filter surface 120S1. The oxidation catalyst filter width 120W may be, for example, a distance between two side surfaces that are connected to the outer periphery of the first oxidation catalyst filter surface 120S1 and face each other.

The shape of each of the first oxidation catalyst filter surface 120S1 and the second oxidation catalyst filter surface 120S2 may be, for example, circular, oval, trigonal, tetragonal, pentagonal, hexagonal, or octagonal, but is not necessarily limited thereto. Any form suitable for the standard in the art may be used. Accordingly, the volume and shape of the oxidation catalyst filter 120 may be defined by, for example, the shape of the first oxidation catalyst filter surface 120S1, the shape of the second oxidation catalyst filter surface 120S2, and the oxidation catalyst filter thickness 120T.

In an embodiment, the oxidation catalyst filter thickness 120T may be smaller than the oxidation catalyst filter width 120W. The oxidation catalyst filter thickness 120T may be, for example, less than or equal to 50 %, less than or equal to 40 %, less than or equal to 30 %, less than or equal to 20 %, less than or equal to 10 %, or less than or equal to 5 % of the oxidation catalyst filter width 120W. The oxidation catalyst filter thickness 120T may be, for example, about 0.1 % to about 50 %, about 0.1 % to about 40 %, about 0.5 % to about 30 %, about 0.5 % to about 20 %, about 1 % to about 10 %, or about 1 % to about 5 %, of the oxidation catalyst filter width 120W. The oxidation catalyst filter thickness 120T may be, for example, about 0.1 mm to about 100 mm, about 0.5 mm to about 70 mm, about 1 mm to about 50 mm, about 5 mm to about 50 mm, or about 10 mm to about 50 mm. When the oxidation catalyst filter 120 has a thickness within these ranges, the air purification module 1000 that includes the oxidation catalyst filter 120 may have a more simplified structure, a further reduced volume, and further improved energy efficiency. When the thickness of the oxidation catalyst filter 120 is too small, the amount of a catalyst included in the oxidation catalyst filter 120 is excessively reduced, so that it may be difficult to effectively purify the air. When the oxidation catalyst filter 120 is too thick, heat released from the light-emitting heat source 200 may not be transferred to the entire oxidation catalyst filter 120.

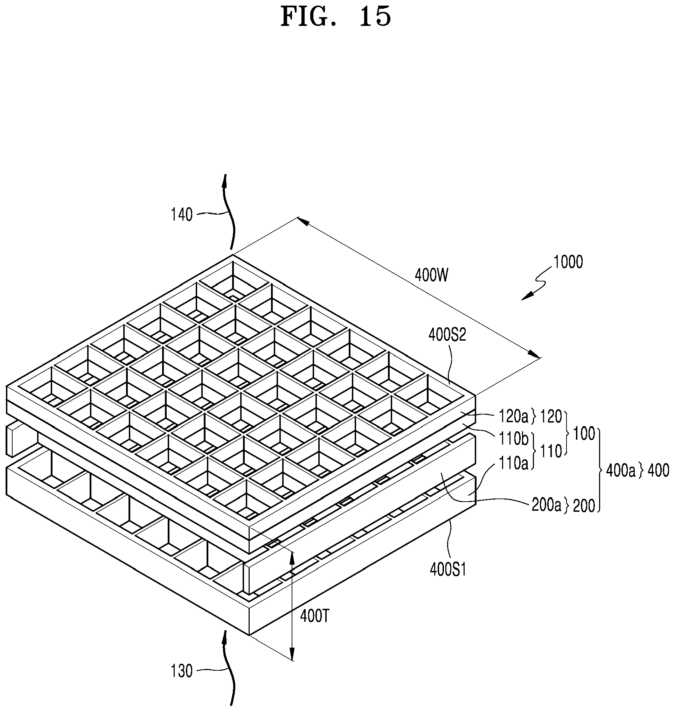

FIG. 15 is a schematic view of the air purification module according to another embodiment.

Referring to FIG. 15, the air purification module 1000 includes an assembly 400 including the catalyst filter 100 and the light-emitting heat source 200. The assembly 400 may include a first assembly 400a. The catalyst filter 100 may include the photocatalyst filter 110 and the oxidation catalyst filter 120. The catalyst filter 100 may include, for example, a 1-1 photocatalyst filter 110a and a 1-1 oxidation catalyst filter 120a. The light-emitting heat source 200 may include a 1-1 light-emitting heat source 200a. The 1-1 photocatalyst filter 110a may be disposed upstream of air flow, compared to the 1-1 light-emitting heat source 200a. The 1-1 oxidation catalyst filter 120a may be disposed downstream of air flow, compared to the 1-1 light-emitting heat source 200a. When the air purification module 1000 includes the first assembly 400a, the air purification module 1000 may have a simplified structure, a reduced volume, and improved energy efficiency.

The first assembly 400a may further include, for example, a 1-2 photocatalyst filter 110b disposed between the 1-1 light-emitting heat source 200a and the 1-1 oxidation catalyst filter 120a. When the first assembly 400a further includes the 1-2 photocatalyst filter 110b, the utilization rate of light emitted from the 1-1 light-emitting heat source 200a may be improved.

Referring to FIG. 15, the assembly 400 may include a first assembly surface 400S1 disposed upstream of air flow and a second assembly surface 400S2 disposed downstream of air flow. The assembly 400 may have, for example, an assembly thickness 400T from first assembly surface 400S1 to second assembly surface 400S2. The assembly 400 may have, for example, an assembly width 400W defined by a distance between opposite outer peripheral ends of the first assembly surface 400S1. The assembly width 400W may be, for example, a distance between two side surfaces that are connected to the outer periphery of the first assembly surface 400S1 and face each other.

The shape of each of the first assembly surface 400S1 and the second assembly surface 400S2 may be, for example, circular, oval, trigonal, tetragonal, pentagonal, hexagonal, or octagonal, but is not necessarily limited thereto. Any form suitable for the standard in the art may be used. Accordingly, the volume and shape of the assembly 400 may be defined by, for example, the shape of the first assembly surface 400S1, the shape of the second assembly surface 400S2, and the assembly thickness 400T.

The assembly thickness 400T may be smaller than the assembly width 400W. The assembly thickness 400T may be, for example, less than or equal to 50 %, less than or equal to 40 %, less than or equal to 30 %, less than or equal to 20 %, or less than or equal to 10 % of the assembly width 400W. The assembly thickness 400T may be, for example, about 1 % to about 50 %, about 1 % to about 40 %, about 5 % to about 30 %, about 5 % to about 20 %, or about 5 % to about 10 %. The assembly thickness 400T may be, for example, about 0.1 mm to about 100 mm, about 0.5 mm to about 70 mm, about 1 mm to about 50 mm, about 5 mm to about 50 mm, or about 10 mm to about 50 mm. When the assembly 400 has a thickness within these ranges, the air purification module 1000 that includes the assembly 400 may have a more simplified structure, a further reduced volume, and further improved energy efficiency. When the assembly 400 is too thin, the amount of a catalyst included in the assembly 400 may be excessively reduced, and thus the air may not be effectively purified. When the assembly 400 is too thick, heat released from the light-emitting heat source 200 may not be transferred to the entire assembly 400.

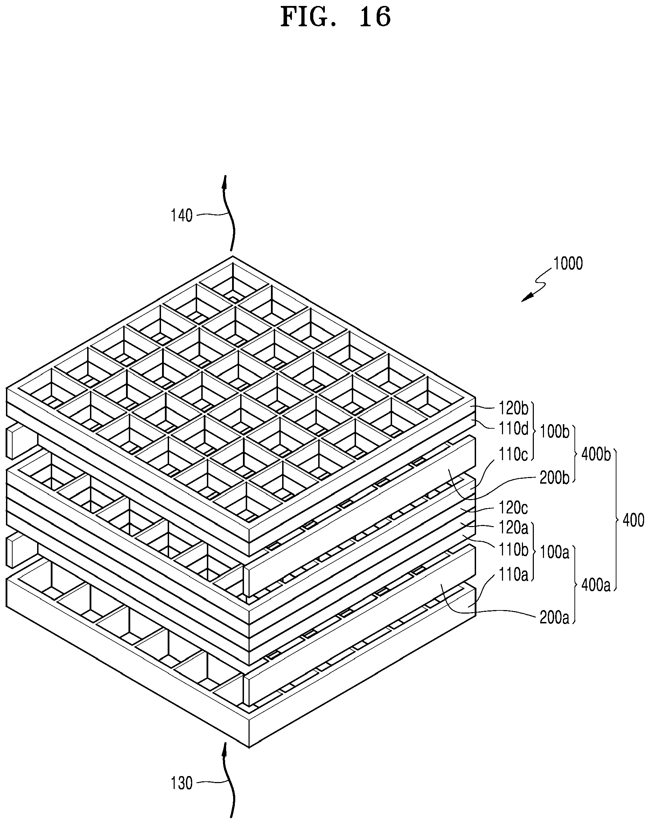

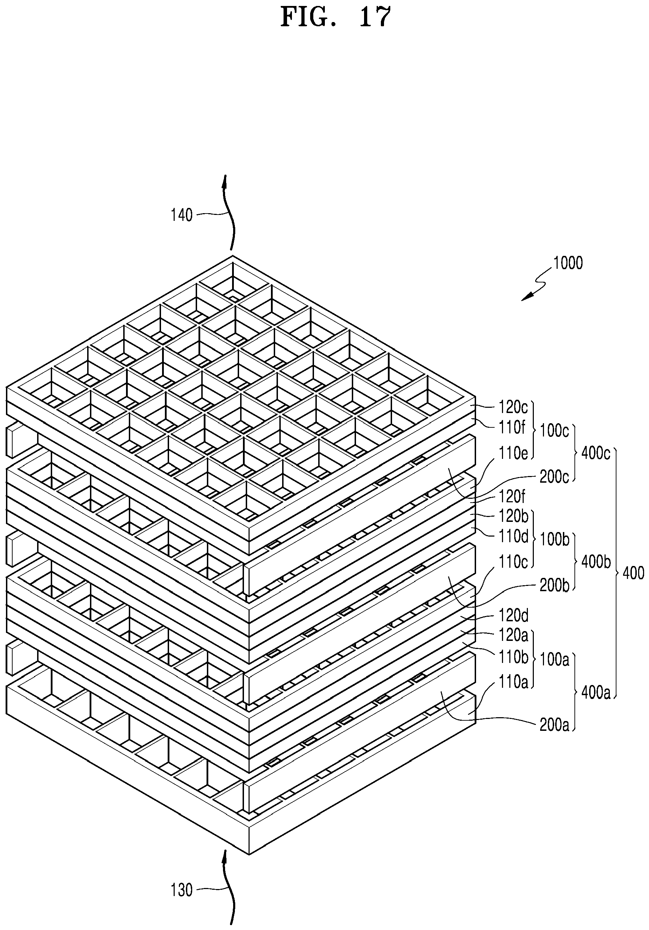

FIG. 16 is a schematic view of the air purification module according to another embodiment. FIG. 17 is a schematic view of the air purification module according to another embodiment.

Referring to FIG. 16, the air purification module 1000 includes the assembly 400. The assembly 400 may include the first assembly 400a and at least one second assembly 400b disposed downstream of air flow, compared to the first assembly 400a. The first assembly 400a has the same configuration as that of FIG. 15. The second assembly 400b may include a 2-1 light-emitting heat source 200b, a 2-1 photocatalyst filter 110c, and a 2-1 oxidation catalyst filter 120b. The 2-1 photocatalyst filter 110a may be disposed upstream of air flow, compared to the 2-1 light-emitting heat source 200b. The 2-1 oxidation catalyst filter 120b may be disposed downstream of air flow, compared to the 2-1 light-emitting heat source 200b. When the air purification module 1000 includes the first assembly 400a and at least one second assembly 400b, the air purification module 1000 may have improved air purification capability.

Referring to FIG. 17, the air purification module 1000 includes the assembly 400, and the assembly 400 may include the first assembly 400a and a plurality of second assemblies 400b and 400c.

The first assembly 400a has the same configuration as that of FIG. 15. In the plurality of second assemblies 400b and 400c, the second assembly 400b adjacent to the first assembly 400a may have the same configuration as that of FIG. 16.

The second assembly 400c spaced apart from the first assembly 400a may be, for example, a third assembly 400c. The third assembly 400c may include a 3-1 light-emitting heat source 200c, a 3-1 photocatalyst filter 110e, and a 3-1 oxidation catalyst filter 120c. The 3-1 photocatalyst filter 110e may be disposed upstream of air flow, compared to the 3-1 light-emitting heat source 200c. The 3-1 oxidation catalyst filter 120c may be disposed downstream of air flow, compared to the 3-1 light-emitting heat source 200c.

The number of second assemblies included in the air purification module 1000 may be, for example, 1 to 1,000, 1 to 500, 1 to 100, 1 to 50, 1 to 20, 1 to 10, or 1 to 5. The number of second assemblies may be selected according to the performance and size of the air purification module 1000.

Referring to FIG. 16, the second assembly 400b may further include, for example, a 2-2 photocatalyst filter 110d disposed between the 2-1 light-emitting heat source 200b and the 2-1 oxidation catalyst filter 120b. When the second assembly 400b further includes the 2-2 photocatalyst filter 110d, the utilization rate of light emitted from the 2-1 light-emitting heat source 200b may be further improved.

Referring to FIG. 17, the third assembly 400c may further include, for example, a 3-2 photocatalyst filter 110f disposed between the 3-1 light-emitting heat source 200c and the 3-1 oxidation catalyst filter 120c. When the third assembly 400c further includes the 3-2 photocatalyst filter 110f, the utilization rate of light emitted from the 3-1 light-emitting heat source 200c may be further improved.

Referring to FIGS. 16 and 17, the air purification module 1000 may further include, for example, a 2-2 oxidation catalyst filter 120c, 120d, or 120f between the first assembly 400a and the second assembly 400b and/or between adjacent assemblies among the at least one second assembly, e.g., between the second assembly 400b and the third assembly 400c. When the air purification module 1000 further includes the 2-2 oxidation catalyst filter 120c, 120d, or 120f, the air purification module 1000 may have further improved air purification capability.

Referring to FIGS. 15 to 17, a heat insulating material (not shown) may be additionally disposed on a portion or all of the side surface of the assembly 400 in the air purification module 1000. When the portion or all of the side surface of the assembly 400 is coated by the heat insulating material, heat released from the light-emitting heat source 200 may be more effectively transferred to the oxidation catalyst filter 120.

Referring to FIGS. 15 to 17, a light reflective material (not shown) may be additionally disposed on a portion or all of the side surface of the assembly 400 in the air purification module 1000. When the portion or all of the side surface of the assembly 400 is coated by the light reflective material, light emitted from the light-emitting heat source 200 may be more effectively transferred to the photocatalyst filter 110.

Referring to FIGS. 15 to 17, a light-reflective insulation material (not shown) may be additionally disposed on a portion or all of the side surface of the assembly 400 in the air purification module 1000. When the portion or all of the side surface of the assembly 400 is coated by the light-reflective insulation material, light and heat from the light-emitting heat source 200 may be more effectively transferred to the photocatalyst filter 110 and the oxidation catalyst filter 120.

The assembly 400 may include at least one selected from the first assembly 400a and at least one second assembly 400b.

Another aspect provides an air purification system including: the air purification module; an air supply module disposed upstream of air flow, compared to the air purification module; and an air exhaust module disposed downstream of air flow, compared to the air purification module. When the air purification system includes, in addition to the air purification module, an air supply module and an air exhaust module, the air purification system may have further improved air purification capability.

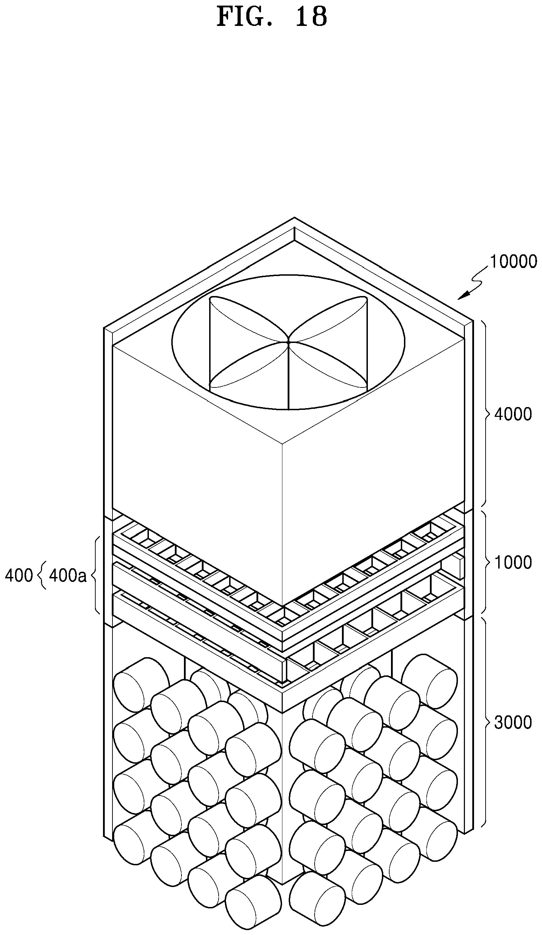

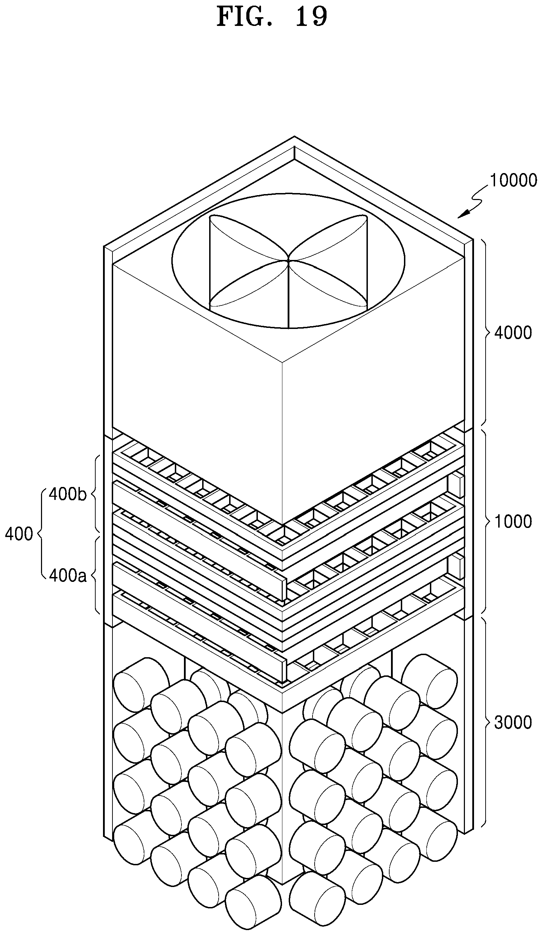

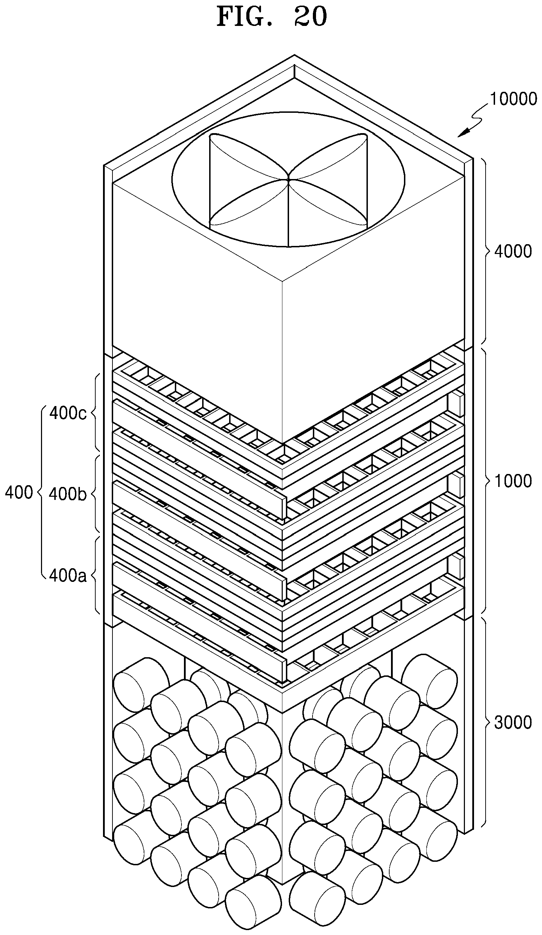

FIG. 18 is a schematic view of the air purification system according to an embodiment. FIG. 19 is a schematic view of the air purification system according to another embodiment. FIG. 20 is a schematic view of the air purification system according to another embodiment.

Referring to FIGS. 18 to 20, the air purification system 10000 includes: the air purification module 1000; an air supply module 3000 disposed upstream of air flow, compared to the air purification module 1000; and an air exhaust module 4000 disposed downstream of air flow, compared to the air purification module 1000.

The air supply module 3000 may provide a means and/or a path for easy transfer of the unpurified air to the air purification module 1000. The air supply module 3000 may include, for example, an air pump and/or an air duct. The air purification module 1000 may further include, for example, a dust collecting filter.

The air exhaust module 4000 may discharge the air purified from the air purification module 1000 to the outside of the air purification system 10000. The air exhaust module 4000 may include, for example, a fan and/or an air duct.

Referring to FIG. 18, the air purification system 10000 includes the air purification module 1000, and the air purification module 1000 includes one assembly 400, for example, the first assembly 400a. The air supply module 3000 may be disposed upstream of air flow, compared to the air purification module 1000, and the air exhaust module 4000 may be disposed downstream of air flow, compared to the air purification module 1000.

Referring to FIG. 19, the air purification system 10000 includes the air purification module 1000, and the air purification module 1000 includes a plurality of assemblies 400, for example, the first assembly 400a and the second assembly 400b. The air supply module 3000 may be disposed upstream of air flow, compared to the air purification module 1000, and the air exhaust module 4000 may be disposed downstream of air flow, compared to the air purification module 1000.

Referring to FIG. 20, the air purification system 10000 includes the air purification module 1000, and the air purification module 1000 includes a plurality of assemblies 400, for example, the first assembly 400a, the second assembly 400b, and the third assembly 400c. The air supply module 3000 may be disposed upstream of air flow, compared to the air purification module 1000, and the air exhaust module 4000 may be disposed downstream of air flow, compared to the air purification module 1000.

Another aspect provides a method of purifying air, the method including: providing a light-emitting heat source; and disposing a catalyst filter, which includes a photocatalyst and an oxidation catalyst, adjacent to the light-emitting heat source, wherein the catalyst filter and the light-emitting heat source may be disposed to purify influent unpurified air and discharge purified air, the light-emitting heat source may irradiate, to the catalyst filter, light for activating the photocatalyst and provide heat to the catalyst filter to activate the oxidation catalyst therein, and the temperature of the light-emitting heat source may be higher than the temperature of the unpurified air. By using one light-emitting heat source, the air may be purified simply, in a reduced space, and with reduced energy, compared to a method using a light source and a heat source separately.

First, the light-emitting heat source may be provided. The light-emitting heat source is not particularly limited, and any source capable of irradiating, to a catalyst filter, light for activating the photocatalyst and providing heat to a catalyst filter to activate an oxidation catalyst therein may be used. The light-emitting heat source may include, for example, ultraviolet LED and/or visible light-emitting LED. The temperature of LED may be, for example, about 20 °C to about 500 °C, about 40 °C to about 300 °C, or about 50°C to about 200 °C. The temperature of the LED may be, for example, higher than the temperature of the unpurified air by at least 20 °C, at least 40 °C, or at least 70 °C.

Next, a catalyst filter including a photocatalyst and an oxidation catalyst may be disposed adjacent to the light-emitting heat source. The catalyst filter may include, for example, a photocatalyst filter and an oxidation catalyst filter. For example, the photocatalyst filter may be disposed upstream of air flow, compared to the light-emitting heat source, and the oxidation catalyst filter may be disposed downstream of air flow, compared to the light-emitting heat source.

The present disclosure is in greater details through Examples and Comparative Examples below. However, the following embodiments are for illustrative purpose only and shall not be construed as limiting the scope of the disclosure.

### (Preparation of air purification module and air purification system)

### Example 1

An air purification system in which an air supply module, an air purification module, and an air exhaust module were sequentially disposed from the upstream to the downstream direction of air flow was prepared.

The air purification module included a first assembly. In the air purification module, a first assembly in which first photocatalyst filter, a light-emitting heat source, a second photocatalyst filter, and an oxidation catalyst filter were sequentially disposed from the upstream to the downstream direction of air flow was prepared.

The volume of the air supply module was 1 cubic meter (m³) (1 m (width) × 1 m (length) × 1 m (height)). The volume of the air exhaust module was 0.5 m³ (1 m × 1 m × 0.5 m). The volume of the air purification module, i.e., the volume of the first assembly, was 0.2 m³ (1 m × 1 m × 0.2 m).

The volume of the air purification system was 1.7 m³.

### Example 2

An air purification system was prepared in the same manner as in Example 1, except that the air purification module additionally included a second assembly disposed downstream of the air flow of the first assembly. The second assembly had the same volume and structure as those of the first assembly.

The volume of the air supply module was 1 m³ (1 m (width) × 1 m (length) × 1 m (height)). The volume of the air exhaust module was 0.5 m³ (1 m × 1 m × 0.5 m). The volume of the air purification module was 0.4 m³.

The volume of the air purification system was 1.9 m³.

### Example 3

An air purification system was prepared in the same manner as in Example 1, except that the air purification module additionally included a second assembly and a third assembly sequentially disposed downstream of the air flow of the first assembly. The second assembly and the third assembly each had the same volume and structure as those of the first assembly.

The volume of the air supply module was 1 m³ (1 m (width) × 1 m (length) × 1 m (height)). The volume of the air exhaust module was 0.5 m³ (1 m × 1 m × 0.5 m). The volume of the air purification module was 0.6 m³.

The volume of the air purification system was 2.1 m³.

### Comparative Example 1

An air purification system in which an air supply module, an air purification module, and an air exhaust module were sequentially disposed from the upstream to the downstream direction of air flow was prepared.

The air purification module included a fourth assembly. In the air purification module, a fourth assembly in which first photocatalyst filter, a light source, a second photocatalyst filter, a heat source, and an oxidation catalyst were sequentially disposed from the upstream to the downstream direction of air flow was prepared.

The volume of the air supply module was 1 m³ (1 m (width) × 1 m (length) × 1 m (height)). The volume of the air exhaust module was 0.5 m³ (1 m × 1 m × 0.5 m). The volume of the air purification module, i.e., the volume of the fourth assembly, was 0.5 m³ (1 m × 1 m × 0.5 m).

The volume of the air purification system was 2.0 m³.

### Comparative Example 2

An air purification system was prepared in the same manner as in Comparative Example 1, except that the air purification module additionally included a fifth assembly disposed downstream of the air flow of the fourth assembly. The fifth assembly had the same volume and structure as those of the fourth assembly.

The volume of the air supply module was 1 m³ (1 m (width) × 1 m (length) × 1 m (height)). The volume of the air exhaust module was 0.5 m³ (1 m × 1 m × 0.5 m). The volume of the air purification module was 1.0 m³.

The volume of the air purification system was 2.5 m³.

### Comparative Example 3

An air purification system was prepared in the same manner as in Comparative Example 1, except that the air purification module additionally included a fifth assembly and a sixth assembly sequentially disposed downstream of the air flow of the fourth assembly. The fifth assembly and the sixth assembly each had the same volume and structure as those of the fourth assembly.

The volume of the air supply module was 1 m³ (1 m (width) × 1 m (length) × 1 m (height)). The volume of the air exhaust module was 0.5 m³ (1 m × 1 m × 0.5 m). The volume of the air exhaust module was 1.5 m³ (1 m × 1 m × 1.5 m).

The volume of the air purification system was 3.0 m³.

### Evaluation Example 1: Comparison of volume and power consumption

The volumes and power consumptions of the air purification systems prepared in Examples 1 to 3 and Comparative Examples 1 to 3 were compared, and the results are shown in Table 1.

**Table 1**

| | Air purification module | air purification system | Power consumption |
|---|---|---|---|
| | Volume [m³] | Volume [m³] | [kWh] |
| Example 1 | 0.2 | 1.7 | 23.8 |
| Example 2 | 0.4 | 1.9 | 47.6 |
| Example 3 | 0.6 | 2.1 | 71.4 |
| Comparative Example 1 | 0.5 | 2.0 | 81.4 |
| Comparative Example 2 | 1.0 | 2.5 | 162.8 |
| Comparative Example 3 | 1.5 | 3.0 | 244.2 |

As shown in Table 1, the volumes of the air purification modules of Examples 1 to 3 were reduced by 50 % or more compared to the volumes of the air purification modules of Comparative Examples 1 to 3.

The volumes of the air purification systems of Examples 1 to 3 were reduced by 15 % or more compared to the volumes of the air purification systems of Comparative Examples 1 to 3.

The power consumptions of the air purification systems of Examples 1 to 3 were reduced by 60 % or more compared to the power consumptions of the air purification systems of Comparative Examples 1 to 3.

Accordingly, it was confirmed that the air purification modules and air purification systems of Examples 1 to 3 provided a simple structure, a reduced volume, and improved energy efficiency compared to the air purification modules and air purification systems of Comparative Examples 1 to 3.

According to the one or more embodiments, an air purification module includes a light-emitting heat source that provides light for activating a photocatalyst and heat for activating an oxidation catalyst at the same time, and thus may have a simplified structure, a compact volume, and improved energy efficiency.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. An air purification module configured to purify influent unpurified air and discharge purified air and comprising:
a catalyst filter including a photocatalyst and an oxidation catalyst; and
a light-emitting heat source disposed adjacent to the catalyst filter,
wherein the light-emitting heat source irradiates, to the catalyst filter, light for activating the photocatalyst and provides heat to the catalyst filter to activate the oxidation catalyst therein, and
a temperature of the light-emitting heat source is higher than a temperature of the unpurified air.

2. The air purification module of claim 1, wherein the temperature of the light-emitting heat source is higher than the temperature of the unpurified air by at least 20 degrees in Celsius (°C), and
the temperature of the light-emitting heat source is 20 °C to 1,000 °C.

3. The air purification module of claim 1 or 2, wherein the light for activating the photocatalyst includes radio waves, microwaves, ultraviolet rays, visible rays, infrared rays, X-rays, or a combination thereof.

4. The air purification module of any preceding claim, wherein the light-emitting heat source includes a first light-emitting heat source surface disposed upstream of air flow and a second light-emitting heat source surface disposed downstream of the air flow,
wherein the light-emitting heat source has a thickness from the first light-emitting heat source surface to the second light-emitting heat source surface and a width defined by a distance between opposite outer peripheral ends of the first light-emitting heat source surface,
the thickness of the light-emitting heat source is less than or equal to 50 % of the width of the light-emitting heat source, and
the thickness of the light-emitting heat source is 1 millimeter (mm) to 200 mm.

5. The air purification module of any of claims 1 to 3, wherein the light-emitting heat source includes a support and at least one source for the light-emitting heat supply disposed on one surface or opposite surfaces of the support,
wherein the at least one source for the light-emitting heat supply emits light in a first direction crossing a major surface of the support,
the at least one source for the light-emitting heat supply releases heat in the first direction and a second direction different from the first direction,
the light-emitting heat source additionally includes a light reflective layer, a heat insulating layer, or a light-reflective and heat-insulating layer, and
the light reflective layer, the heat insulating layer, or the light-reflective and heat-insulating layer is disposed on at least one selected from an inner side surface of the light-emitting heat source and the opposite surfaces of the support.

6. The air purification module of any preceding claim, wherein the air purification module includes only the light-emitting heat source as an energy source,
a first volume of the air purification module is smaller than a second volume of an air purification module including energy sources other than the light-emitting heat source,
the first volume is 50 % of less of the second volume,
a first power consumption of the air purification module including the light-emitting heat source is smaller than a second power consumption of the air purification module including the energy sources other than the light-emitting heat source, and
the first power consumption is 50 % less of the second power consumption.

7. The air purification module of any preceding claim, wherein the catalyst filter includes
a first catalyst filter surface disposed upstream of air flow and a second catalyst filter surface disposed downstream of the air flow,
the catalyst filter has a thickness from the first catalyst filter surface to the second catalyst filter surface and a width defined by a distance between opposite outer peripheral ends of the first catalyst filter surface,
the thickness of the catalyst filter is less than or equal to 50 % of the width of the catalyst filter,
the thickness of the catalyst filter is 1 mm to 200 mm,
the air purification module additionally includes a light reflective layer, a heat insulating layer, or a light-reflective and heat insulation layer, and
the light reflective layer, the heat insulating layer, or the light-reflective and heat insulation layer is disposed on an inner surface of the catalyst filter, and optionally wherein the catalyst filter includes
a plurality of cells forming an air flow path along a thickness direction of the catalyst filter and disposed parallel and adjacent to each other, and optionally wherein the plurality of cells disposed parallel are disposed regularly or irregularly, or
the plurality of cells disposed parallel are disposed periodically or non-periodically.

8. The air purification module of claim 7, wherein the plurality of cells disposed along the thickness direction of the catalyst filter include through-holes and partition walls defining a shape of the through-holes, and
the plurality of cells disposed along the thickness direction of the catalyst filter include a protrusion or a depression, and optionally wherein an inlet of the through-hole is circular, oval, trigonal, tetragonal, pentagonal, hexagonal, octagonal, or a combination thereof in a view from the thickness direction of the catalyst filter,
a surface of the protrusion is circular, oval, trigonal, tetragonal, pentagonal, hexagonal, octagonal, or a combination thereof,
a bottom portion of the protrusion is circular, oval, trigonal, tetragonal, pentagonal, hexagonal, octagonal, or a combination thereof in the view from the thickness direction of the catalyst filter,
a diameter of the through-hole increases, decreases, or remains constant along the thickness direction of the catalyst filter,
a diameter of the protrusion increases, decreases, or remains constant along the thickness direction of the catalyst filter, or
a diameter of the depression increases, decreases, or remains constant along the thickness direction of the catalyst filter.

9. The air purification module of any preceding claim, wherein the catalyst filter includes a solid substrate and a catalyst coated on the solid substrate,
wherein the catalyst includes a photocatalyst and an oxidation catalyst, and optionally wherein: the photocatalyst includes a first metal, a first metal oxide, a first metal carbide, a first metal nitride, a first metal oxynitride, or a combination thereof;
the first metal includes Ti, Zn, Zr, Ta, Nb, W, Cu, Pt, Au, Ag, Zn, Pd, an alloy thereof, or a combination thereof;
the photocatalyst is in a form of a particle, and the particle has a spherical shape, a tube shape, a rod shape, a fiber shape, a sheet shape, or a combination thereof; and
the particle is a primary particle or a secondary particle that is a combination of a plurality of primary particles, and further optionally wherein: the oxidation catalyst includes a second metal, a second metal oxide, a second metal carbide, a second metal nitride, a second metal oxynitride, or a combination thereof;
the second metal is different from the first metal, and includes Ti, Mn, Co, Ce, Al, Fe, Ni, Na, In, Bi, W, Sn, Pt, Au, Ag, Zn, Pd, an alloy thereof, or a combination thereof;
the oxidation catalyst is in a form of a particle, and the particle has a spherical shape, a tube shape, a rod shape, a fiber shape, a sheet shape, or a combination thereof; and
the particle of the oxidation catalyst is a primary particle or a secondary particle that is a combination of a plurality of primary particles.

10. The air purification module of any preceding claim, wherein the catalyst filter includes:
a photocatalyst filter disposed adjacent to the light-emitting heat source and including the photocatalyst; and
an oxidation catalyst filter disposed adjacent to the light-emitting heat source and including the oxidation catalyst, and optionally wherein the photocatalyst filter is disposed upstream or downstream of air flow, compared to the light-emitting heat source, and
the oxidation catalyst filter is disposed downstream of the air flow, compared to the light-emitting heat source.

11. The air purification module of claim 10, wherein the photocatalyst filter includes a first photocatalyst filter surface disposed upstream of air flow and a second photocatalyst filter surface disposed downstream of the air flow,
wherein the photocatalyst filter has a thickness from the first photocatalyst filter surface to the second photocatalyst filter surface and a width defined by a distance between opposite outer peripheral ends of the first photocatalyst filter surface,
the thickness of the photocatalyst filter is 50 % or less of the width of the photocatalyst filter, and
the thickness of the photocatalyst filter is 0.1 mm to 100 mm.

12. The air purification module of any preceding claim, further comprising an assembly including the catalyst filter and the light-emitting heat source,
wherein the catalyst filter includes a 1-1 photocatalyst filter and a 1-1 oxidation catalyst filter,
the light-emitting heat source includes a 1-1 light-emitting heat source,
the 1-1 photocatalyst filter is disposed upstream of air flow, compared to the 1-1 light-emitting heat source, and
the 1-1 oxidation catalyst filter is disposed downstream of the air flow, compared to the 1-1 light-emitting heat source, and optionally further comprising a 1-2 photocatalyst filter disposed between the 1-1 light-emitting heat source and the 1-1 oxidation catalyst filter.

13. The air purification module of claim 12, wherein the assembly includes a first assembly and at least one second assembly disposed downstream of the air flow, compared to the first assembly,
the at least second assembly includes a 2-1 light-emitting heat source, a 2-1 photocatalyst filter, and a 2-1 oxidation catalyst filter,
the 2-1 photocatalyst filter is disposed upstream of the air flow, compared to the 2-1 light-emitting heat source,
the 2-1 oxidation catalyst filter is disposed downstream of the air flow, compared to the 2-1 light-emitting heat source, and
a total number of the at least one second assembly is 1 to 1,00, and optionally further comprising a 2-2 photocatalyst filter disposed between the 2-1 light-emitting heat source and the 2-1 oxidation catalyst filter.

14. An air purification system comprising:
the air purification module of any preceding claim;
an air supply module disposed upstream of air flow, compared to the air purification module; and
an air exhaust module disposed downstream of the air flow, compared to the air purification module.

15. A method of purifying air, the method comprising:
providing a light-emitting heat source; and
disposing a catalyst filter, which includes a photocatalyst and an oxidation catalyst, adjacent to the light-emitting heat source,
wherein the catalyst filter and the light-emitting heat source are configured to purify influent unpurified air and discharge purified air,
the light-emitting heat source irradiates, to the catalyst filter, light for activating the photocatalyst and provides heat to the catalyst filter to activate the oxidation catalyst therein, and
a temperature of the light-emitting heat source is higher than a temperature of the unpurified air.
